# FASCICULE DE BREVET EUROPEEN

(11) **EP 3 692 017 B1**
(45) Date de publication et mention de la délivrance du brevet: **29.11.2023**
(21) Numéro de dépôt: 18864901.6
(22) Date de dépôt: 02.10.2018
(51) Int. Cl.: C07D 209/48, C08G 75/14, H01M 10/056, H01M 4/137, C08G 73/00

(54) **POLYMÈRES ET COMPOSÉS SOUFRÉS ET LEUR UTILISATION DANS LES CELLULES ÉLECTROCHIMIQUES**
SCHWEFELHALTIGE VERBINDUNGEN UND POLYMERE UND DEREN VERWENDUNG IN ELEKTROCHEMISCHEN ZELLEN
SULFUR-CONTAINING COMPOUNDS AND POLYMERS AND THE USE THEREOF IN ELECTROCHEMICAL CELLS

(30) Priorité: 02.10.2017 CA 2981012
(43) Date de publication de la demande: 12.08.2020
(73) Titulaire: Hydro-Québec, Montréal, QC H2Z 1A4 (CA)
(72) Inventeur: LÉVESQUE-BÉLANGER, Rachel, Mercier, Québec J6R 1X9 (CA); PAOLELLA, Andrea, Montréal, Québec H2W 2J4 (CA); DAIGLE, Jean-Christophe, St-Bruno-De-Montarville, Québec J3V 1V7 (CA); COMMARIEU, Basile, Montréal, Québec H2W 2M4 (CA); ARMAND, Michel, 75014 Paris (FR); ZAGHIB, Karim, Longueuil, Québec J4N 1T8 (CA)
(74) Mandataire: Lavoix
(86) Numéro de dépôt international: PCT/CA2018/051239
(87) Numéro de publication internationale: WO 2019/068182

(56) Documents cités:
- EP-A1- 0 387 106
- EP-A2- 0 213 573
- EP-A2- 0 449 117
- WO-A1-99/54363
- CA-A- 1 123 543
- DE-A1- 4 243 463
- DE-A1- 4 243 463
- US-A- 4 716 216
- GASPERT, B. et al.: "Optically Active Trisulphides and Tetrasulphides Related to L-Cysteine", Croatica Chemica Acta, vol. 32, 13 May 1960 (1960-05-13), pages 85-90, XP055588797,
- BUCK, M. et al.: "Non-Destructive In Situ Analysis of Interface Processes and Thin Filn Growth", J. Adhesion, vol. 58 , pages 227-241,
- YORIFUJI DAISUKE ET AL: "Molecular Structure Dependence of Out-of-Plane Thermal Diffusivities in Polyimide Films: A Key Parameter for Estimating Thermal Conductivity of Polymers", MACROMOLECULES, AMERICAN CHEMICAL SOCIETY, US , vol. 43, no. 18 28 September 2010 (2010-09-28), pages 7583-7593, XP009527490, ISSN: 0024-9297, DOI: 10.1021/MA101066P Retrieved from the Internet: URL:https://pubs.acs.org/doi/abs/10.1021/m a101066p

## Description

### DEMANDE RELIÉE

La présente demande revendique la priorité, sous la loi applicable, de la demande de brevet canadienne no 2,981,012 déposée le 2 octobre 2017.

### DOMAINE TECHNIQUE

La présente demande se rapporte au domaine des polymères et oligomères pour utilisation comme matériau électrochimiquement actif d'électrode positive, comme électrolyte polymère solide (SPE), ou comme additif pour électrolyte, notamment dans les piles au lithium.

### ÉTAT DE LA TECHNIQUE

Une prise de conscience environnementale globale influence un grand nombre de scientifiques afin de trouver des réponses à la problématique des gaz à effet de serre et à leurs impacts sur notre planète. Sachant que les transports jouent un rôle majeur dans l'émission des gaz à effet de serre, l'électrification des transports fait définitivement partie de la solution au réchauffement planétaire. Il est alors important de concevoir de nouveaux matériaux de batteries plus verts, abordables, sécuritaires et permettant des performances se rapprochant de celles des combustibles fossiles (telles que l'autonomie et la puissance).

Les cathodes organiques sont des candidats de choix pour la confection de batteries pouvant répondre à la demande en ce qui a trait au stockage d'énergie. Un avantage de ces matériaux se situe au niveau de la température de synthèse qui est beaucoup plus basse que celle des matériaux d'intercalation (Armand et al., Nature, 2008, 451, 652). De plus, elles sont composées d'éléments abondants et proposent d'excellentes performances électrochimiques (Le Gall et al., Journal of Power Sources, 2003, 316-320). Par contre, leur plus grand désavantage concerne la dissolution du matériau actif dans l'électrolyte, ce qui affecte grandement la stabilité de la batterie (Chen et al., ChemSusChem 2008, 1, 348). Les polymères organiques électrochimiquement actifs peuvent permettre de contrer ou réduire la dissolution du matériau actif pour l'obtention d'un matériau de plus grande cyclabilité (Zeng et al. Electrochimica Acta, 2014, 447-454).

Le soufre élémentaire est aussi un candidat pour la confection de batteries possédant une grande densité énergétique. En effet, les batteries lithium-soufre (Li-S) sont très prometteuses compte tenu des avantages qu'offre cet élément, c'est-à-dire une capacité élevée de 1675 mAhg⁻¹ et une grande abondance, celle-ci étant due au fait que le soufre est un produit secondaire du raffinage du pétrole (Hyun et al., ACS Energy Lett. 2016, 1, 566-572). Par contre, les batteries Li-S présentent également plusieurs inconvénients, dont la dissolution des polysulfures (Li₂Sₓ, x=4-8) produits lors de la réduction du soufre durant le cyclage. De plus, une fois dissouts, les polysulfures sont impliqués dans un phénomène appelé « *shuttle effect »* qui déstabilise la surface du lithium (anode). Ce phénomène entraine une réduction de la stabilité ainsi qu'une faible efficacité coulombique (Zhou et al. J. Am. Chem. Soc. 2013, 135, 16736-16743).

Une stratégie employée pour solutionner ce problème comprend l'encapsulation des particules de soufre. Par exemple, Zhou et al. (J. Am. Chem. Soc. 2013, 135, 16736-16743) ont effectué l'enrobage du soufre avec de la polyaniline (PANI) afin d'immobiliser les polysulfures à l'intérieur de cette coquille. L'encapsulation du soufre permet alors d'augmenter la stabilité de la batterie. Par contre, l'ajout de cet élément limite la quantité de soufre dans le matériau actif à seulement 58% en masse, ce qui signifie que 42% en poids du matériau est constitué de matière électrochimiquement inactive, ceci réduisant la densité de charge du matériau d'électrode. Malgré que cette stratégie permette tout de même d'obtenir une efficacité coulombique supérieure à 97%, elle présente toujours une cyclabilité limitée à long terme.

La synthèse de composites carbone-soufre a aussi été présentée. Par exemple, Wang et al., (Journal of Power Sources, 2011, 7030-7034) ont effectué la synthèse d'un composite graphène-soufre. Ce nouveau matériau permet d'augmenter non seulement la conductivité intrinsèque, mais permet également d'atteindre une capacité initiale presque équivalente à la valeur théorique. Par contre, la présence de graphène ne permet pas de contrer le phénomène de dissolution des polysulfures, ce matériau présentant une perte de capacité équivalente à celle du soufre lui-même.

La copolymérisation du soufre élémentaire avec un ou plusieurs monomères organiques a aussi été explorée afin d'obtenir un polymère riche en soufre. Lorsque le soufre est chauffé au-delà de 159°C, il y a ouverture du cycle (S₈) formant ainsi un diradical pouvant polymériser de façon radicalaire. Chung et al., (Nature chemistry, 2013,5, 518-524) ont développé un copolymère riche en soufre pour les batteries au Li-S. Par vulcanisation inverse, le soufre élémentaire a été copolymérisé avec différents pourcentages de 1,3-diisopropénylbenzène (DIB). La copolymérisation permet de créer une matrice pouvant limiter la dissolution des polysulfures. Leurs résultats électrochimiques présentent une grande efficacité coulombienne ainsi qu'une bonne rétention de la capacité. Par contre, comme présenté précédemment, le monomère organique utilisé est électrochimique non actif, ce qui entraîne la présence d'une masse inerte et par conséquent une diminution de la densité énergétique.

Par conséquent, il existe donc un besoin pour le développement de nouveaux matériaux d'électrode, par exemple, combinant certains avantages des matériaux précédents tout en excluant au moins l'un de leurs désavantages.

### SOMMAIRE

Selon un premier aspect, la présente description concerne un polymère de Formule I : 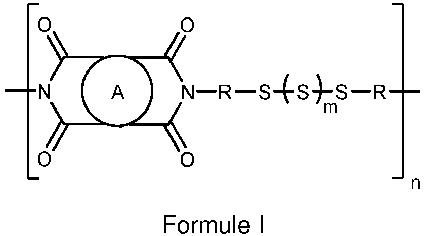 dans laquelle :
A est choisi parmi les groupements insaturés permettant la délocalisation des électrons, par exemple, les groupements aryles et hétéroaryles substitués ou non substitués, et leurs équivalents polycycliques fusionnés ou non;
R est un groupement organique de liaison substitué ou non substitué choisi parmi C₂₋₆alkylène linéaire ou ramifié, C₂₋₆alkylèneoxy linéaire ou ramifié, C₂₋₆alkylèneglycol linéaire ou ramifié, C₂₋₆alkylèneoxyC₂₋₆alkylène linéaire ou ramifié, poly(C₂₋₆alkylèneglycol) linéaire ou ramifié, C₆₋₁₂arylène, C₃₋₁₂cycloalkylène, C₅₋₁₂hétéroarylène, C₃₋₁₂hétérocycloalkylène;
m représente le nombre moyen d'atomes de soufre insérés dans le pont disulfure des maillons du polymère et ne peut être nul, c'est-à-dire m > 0, par exemple 0 < m ≤ 8, ou 1 ≤ m ≤ 6, ou 1 ≤ m ≤ 4; et
n représente le nombre moyen d'unités dans le polymère, par exemple, n peut être situé dans l'intervalle de 2 à 500, ou de 5 à 300.

Selon un mode de réalisation, A est choisi parmi les groupements benzène, naphtalène, pérylène, et biphényle. Par exemple, A est un groupement benzène, le polymère étant de Formule I(a) : 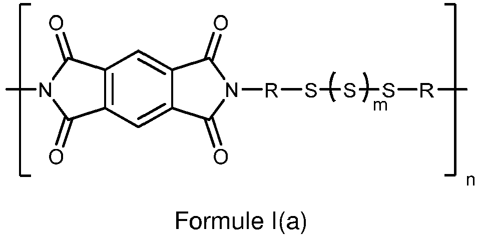 dans lequel R, m et n sont tels qu'ici définis.

Selon un autre mode de réalisation, R est choisi parmi les groupements benzène, éthylène, propylène, poly(éthylène glycol), poly(propylène glycol), et les copolymères d'éthylène glycol et de propylène glycol.

Un exemple de polymère est aussi illustré par la Formule I(b): 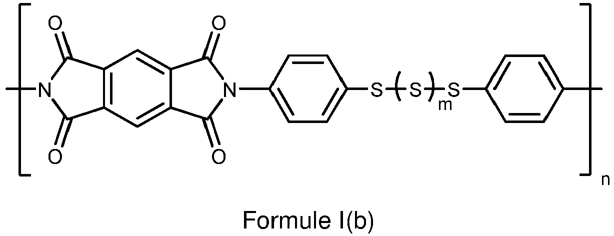 dans laquelle m et n sont tels qu'ici définis.

Selon un autre aspect, la présente description concerne un composé de Formule Il : 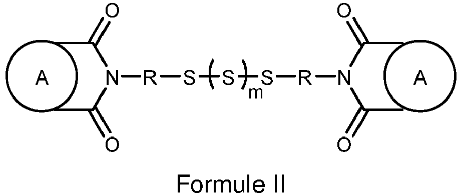 dans laquelle A et R sont tels qu'ici définis, et m représente le nombre d'atomes de soufre insérés dans le pont disulfure du composé, par exemple, m > 0, par exemple 0 < m ≤ 8, ou 1 ≤ m ≤ 6, ou 1 ≤ m ≤ 4.

Un exemple de ce composé est représenté par la Formule II(a): 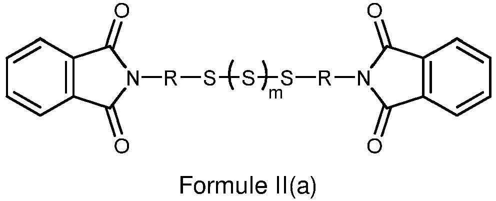 dans laquelle R et m sont tels qu'ici définis.

Un autre exemple du composé est illustré par Formule II(b): 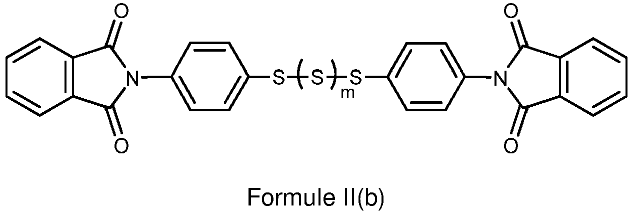 dans laquelle m est tel qu'ici défini.

Selon un autre aspect, la présente description se rapporte à un matériau d'électrode comprenant un polymère ou un composé tels qu'ici définis. Par exemple, le matériau d'électrode comprend en outre un matériau conducteur, un liant, ou une combinaison des deux. Le matériau d'électrode peut aussi contenir du soufre élémentaire libre (Sₓ).

Selon un mode de réalisation, le matériau conducteur est choisi parmi le noir de carbone, le carbone Ketjen^{MC}, le carbone Shawinigan, le noir d'acétylène, le graphite, le graphène, les fibres de carbone (telles les nanofibres de carbone (par exemple, VGCF formé en phase gazeuse)), et les nanotubes de carbone, ou une combinaison d'au moins deux de ceux-ci.

Selon un mode de réalisation, le liant est un liant polymère de type polyéther, polymère fluoré, ou un liant soluble dans l'eau. Selon un exemple, le liant polymère de type polyéther est linéaire, ramifié et/ou réticulé et est basé sur le poly(oxyde d'éthylène) (PEO), le poly(oxyde de propylène) (PPO) ou d'un mélange des deux (ou un co-polymère EO/PO), et comprend éventuellement des unités réticulables. Selon un autre exemple, le liant polymère fluoré est le PVDF (fluorure de polyvinylidène) ou le PTFE (polytétrafluoroéthylène). Enfin, des exemples de liants solubles dans l'eau incluent le SBR (caoutchouc styrène-butadiène), le NBR (caoutchouc acrylonitrile-butadiène), le HNBR (NBR hydrogéné), le CHR (caoutchouc d'épichlorohydrine), ou ACM (caoutchouc d'acrylate), comprenant éventuellement du CMC (carboxyméthylcellulose). Selon un aspect additionnel, la présente description concerne aussi une électrode positive comprenant un matériau d'électrode tel qu'ici défini, appliqué sur un collecteur de courant.

Selon un aspect additionnel, la présente description concerne aussi un électrolyte comprenant un polymère tel qu'ici défini ou un composé tel qu'ici défini. Selon un mode de réalisation, l'électrolyte est un électrolyte liquide comprenant un sel dans un solvant. Selon une alternative, l'électrolyte est un électrolyte en gel comprenant un sel dans un solvant et optionnellement un polymère solvatant. Selon une seconde alternative, l'électrolyte est un électrolyte polymère solide (SPE) comprenant un sel dans un polymère solvatant. Par exemple, le polymère tel qu'ici défini ou un composé tel qu'ici défini est un additif. Alternativement, le polymère solvatant du SPE est le polymère tel qu'ici défini ou un composé tel qu'ici défini. Selon un autre mode de réalisation, le sel est un sel de lithium. Selon un mode de réalisation, l'électrolyte comprend en outre du soufre élémentaire, un liant, un additif ou une combinaison d'au moins deux de ceux-ci.

Un autre aspect se réfère aux cellules électrochimiques comprenant une cathode, un électrolyte et une anode, dans laquelle la cathode comprend un matériau d'électrode tel qu'ici défini. Selon une alternative, ces cellules électrochimiques comprennent une électrode négative, un électrolyte et une électrode positive telle qu'ici définie. Selon une alternative, ces cellules électrochimiques comprennent une cathode, une anode et un électrolyte tel qu'ici défini. La présente description se réfère aussi aux piles au lithium comprenant une telle cellule électrochimique.

En dernier lieu, la présente description se réfère aux procédés de fabrication des polymères et composés ici définis, des matériaux d'électrodes, matériaux d'électrolytes, des électrodes, des électrolytes et des cellules électrochimiques les comprenant. L'utilisation de ces cellules électrochimiques est aussi envisagée, plus particulièrement dans des appareils nomades, par exemple les téléphones portables, les appareils photo, les tablettes ou les ordinateurs portables, dans des véhicules électriques ou hybrides, ou dans le stockage d'énergie renouvelable.

### BRÈVE DESCRIPTION DES FIGURES

La Figure 1 présente les résultats de capacité (mAh/g) et de pourcentage de rétention de capacité en fonction du nombre de cycles en (A) pour la Cellule 1 (référence) incluant le disulfure de polyimide et en (B) pour la Cellule 2 incluant le polyimide-co-polysulfure incluant 35% en poids de soufre.
La Figure 2 montre une courbe galvanostatique présentant les résultats de capacité (mAh/g) en fonction du voltage (V) pour le premier cycle en C/10 de la Cellule 2 incluant le copolymère présenté à l'Exemple 1 (c).
La Figure 3 montre les courbes galvanostatiques présentant les résultats de capacité (mAh/g) en fonction du voltage (V) du premier cycle de charge-décharge pour la Cellule 3 (ligne pointillée), la Cellule 4 (ligne pleine épaisse) et la Cellule 5 (ligne pleine mince).
La Figure 4 montre les courbes galvanostatiques présentant les résultats de capacité (mAh/g) en fonction du voltage (V) du premier cycle de charge-décharge pour la Cellule 6 (ligne pointillée) et pour la Cellule 7 (ligne pleine).
La Figure 5 (A) montre les courbes de cyclovoltammétrie des Cellules 8 (ligne pointillée) et 9 (ligne pleine) incluant le matériau actif de l'Exemple 1 (h) et la Figure 5 (B) montre les courbes galvanostatiques présentant les résultats de capacité (mAh/g) en fonction du voltage (V) du premier cycle de charge-décharge des Cellules 8 et 9.
La Figure 6 présente les spectres de transmission infrarouge des matériaux actifs d'électrode présentés aux Exemples 1 (b), (c), (d), des monomères utilisés lors de la copolymérisation, du copolymère imide sans ajout de soufre et du soufre élémentaire.
La Figure 7 montre les spectres de transmission infrarouge des matériaux actifs d'électrode présentés aux Exemples 1 (f) et 1 (g), des monomères utilisés lors de la copolymérisation, du soufre élémentaire et du copolymère sans ajout de soufre.
La Figure 8 montre les spectres de transmission infrarouge du matériau actif présenté à l'Exemple 1 (h), des monomères utilisés lors de la copolymérisation, du soufre élémentaire et du copolymère sans ajout de soufre.
La figure 9 montre les résultats de conductivité ionique (S cm⁻¹) pour le copolymère présenté à l'Exemple 1 (c) en fonction de la température (K⁻¹).
La figure 10 montre les résultats de conductivité ionique (S cm⁻¹) pour le copolymère présenté à l'Exemple 1 (d) en fonction de la température (K⁻¹).
La figure 11 montre les résultats de conductivité ionique (S cm⁻¹) pour le copolymère présenté à l'Exemple 1 (g) en fonction de la température (K⁻¹). Les valeurs de conductivité ionique pour un film tel que décrit à l'Exemple 5 (c) sont présentées en (A); et les valeurs de conductivité ionique sous forme de poudre pressée tel que décrit à l'Exemple 5 (d) sont présentées en (B).

### DESCRIPTION DÉTAILLÉE

Tous les termes et expressions techniques et scientifiques utilisés ici ont les mêmes définitions que celles généralement comprises par la personne versée dans l'art de la technologie actuelle. La définition de certains termes et expressions utilisés est néanmoins fournie ci-dessous.

Le terme « environ » tel qu'utilisé dans le présent document signifie approximativement, dans la région de, et autour de. Lorsque le terme « environ » est utilisé en lien avec une valeur numérique, il la modifie, par exemple, au-dessus et en dessous par une variation de 10% par rapport à la valeur nominale. Ce terme peut aussi tenir compte, par exemple, de l'erreur expérimentale d'un appareil de mesure ou de l'arrondissement.

Lorsqu'un intervalle de valeurs est mentionné dans la présente demande, les bornes inférieures et supérieures de l'intervalle sont, à moins d'indications contraires, toujours incluses dans la définition.

Les structures chimiques décrites ici sont dessinées suivant les conventions du domaine. Aussi, lorsqu'un atome, comme un atome de carbone, tel que dessiné semble inclure une valence incomplète, alors on assume que la valence est satisfaite par un ou plusieurs atomes d'hydrogène même s'ils ne sont pas explicitement dessinés.

Tels qu'ici utilisés, les termes « alkyle » ou « alkylène » réfèrent à des groupements hydrocarbones saturés ayant entre un et seize atomes de carbone, incluant les groupements linéaires ou ramifiés. Des exemples de groupements alkyles comprennent, sans limitation, méthyle, éthyle, propyle, butyle, pentyle, hexyle, heptyle, octyle, nonyle, décyle, isopropyle, *tert*-butyle, *sec*-butyle, isobutyle, et ainsi de suite. Lorsque le groupement alkyle est localisé entre deux groupements fonctionnels, alors le terme alkylène peut également être utilisé, tel que méthylène, éthylène, propylène, et ainsi de suite. Les termes « Cᵢ-Cᵢᵢalkyle » et « Cᵢ-Cᵢᵢalkylène » réfèrent respectivement à un groupement alkyle ou alkylène ayant du nombre « i » au nombre « ii » d'atome(s) de carbone.

Tel qu'ici utilisés, les termes « aryle » ou « arylène » réfèrent à des groupements aromatiques possédant 4n+2 électrons π(pi), où n est un nombre entier allant de 1 à 3, dans un système conjugué monocyclique ou polycyclique (fusionné ou non) et ayant de six à 20 atomes de cycles. Un système polycyclique inclut au moins un cycle aromatique. Le groupement peut être lié directement, ou connecté via un groupement C₁-C₃alkyle. Des exemples de groupements aryles incluent, sans limitation, phényle, benzyle, phénéthyle, 1-phényléthyl, tolyle, naphtyle, biphényle, terphényle, indényle, benzocyclooctényle, benzocycloheptényle, azulényle, acénaphthylényle, fluorényle, phénanthrényle, anthracényle, pérylényle, et ainsi de suite. Lorsque le groupement aryle est localisé entre deux groupements fonctionnels, alors le terme arylène peut également être utilisé. Le terme aryle ou arylène inclut les groupements substitués ou non substitués. Par exemple, le terme "C₆-Cₙaryle" réfère à un groupe aryles ayant de 6 au nombre "n" indiqué d'atomes de carbone dans la structure cyclique.

Le terme "substitué", lorsqu'inclut en association avec un groupement réfère à un groupement où au moins un atome d'hydrogène a été remplacé par un substituant approprié. Des exemples non-limitatifs de substituants comprennent cyano, halogène (c'est-à-dire F, Cl, Br, ou I), amide, nitro, trifluorométhyle, alkyle inférieur, cycloalkyle, hétérocycloalkyle, aryle, hétéroaryle, alkoxy inférieur, aryloxy, benzyloxy, benzyle, alkoxycarbonyle, sulfonyle, sulfonate, silane, siloxane, phosphonato, phosphinato, et ainsi de suite. Ces substituants peuvent aussi être substitués si permissible, par exemple, si le celui-ci contient un groupement alkyle, alkoxy, aryle, *etc.*

La présente description concerne la copolymérisation du soufre élémentaire avec un monomère organique ou la réaction du soufre avec un composé comprenant un pont disulfure. Par exemple, la présente demande comprend un polymère, celui-ci étant le résultat de la copolymérisation du soufre avec un polyimide électrochimiquement actif. Dans l'alternative, la réaction du soufre se fait avec un disulfure de diimide, obtenant ainsi un composé comprenant une partie sulfurée et une partie organique, les deux étant électrochimiquement actives. Non seulement ces composés et polymères permettront d'améliorer les performances électrochimiques du soufre en réduisant ou en éliminant les problèmes de dissolution, ils pourraient également contribuer à la capacité étant donné la présence de la partie organique, celle-ci étant aussi active en oxydoréduction. Le matériau d'électrode comprenant le polymère ou composé ici décrit peut donc être qualifié de matériau d'électrode positive hybride organo-soufre.

Par exemple, le polymère est un copolymère composé d'un segment polyimide électrochimiquement actif et d'un segment polysulfure de forme -S-(S)ₘ-S- (où m ≥ 1). Un exemple de polymère est représenté par la Formule I : 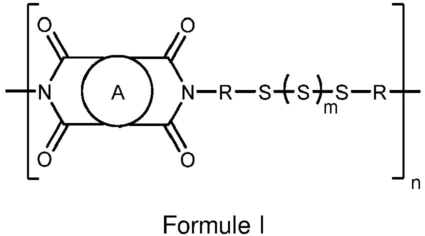 dans laquelle :
A est choisi parmi les groupements insaturés permettant la délocalisation des électrons, par exemple, les groupements aryles et hétéroaryles substitués ou non substitués, et leurs équivalents polycycliques fusionnés ou non;
R est un groupement organique de liaison substitué ou non substitué choisi parmi C₂₋₆alkylène linéaire ou ramifié, C₂₋₆alkylèneoxy linéaire ou ramifié, C₂₋₆alkylèneglycol linéaire ou ramifié, C₂₋₆alkylèneoxyC₂₋₆alkylène linéaire ou ramifié, poly(C₂₋₆alkylèneglycol) linéaire ou ramifié, C₆₋₁₂arylène, C₃₋₁₂cycloalkylène, C₅₋₁₂hétéroarylène, C₃₋₁₂hétérocycloalkylène;
m représente le nombre moyen d'atomes de soufre insérés dans le pont disulfure des maillons du polymère et ne peut être nul, c'est-à-dire m > 0, par exemple 0 < m ≤ 8, ou 1 ≤ m ≤ 6, ou 1 ≤ m ≤ 4; et
n représente le nombre moyen d'unités dans le polymère, par exemple, n peut être situé dans l'intervalle de 2 à 500, ou de 5 à 300.

Selon un exemple, A peut être choisi parmi les groupements benzène, naphtalène, pérylène, et biphényle.

Par exemple, A est un groupement benzène et le polymère est de Formule I(a) : 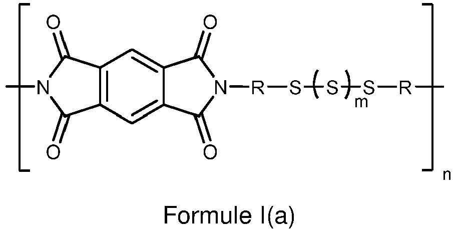 dans laquelle R, m et n sont tels que définis ici.

Selon un autre exemple, R peut être choisi parmi les groupements benzène, éthylène, propylène, poly(éthylène glycol), poly(propylène glycol), et les copolymères d'éthylène glycol et de propylène glycol.

Par exemple, A et R sont des groupements benzène et le polymère est de Formule I(b) ou I(c) : 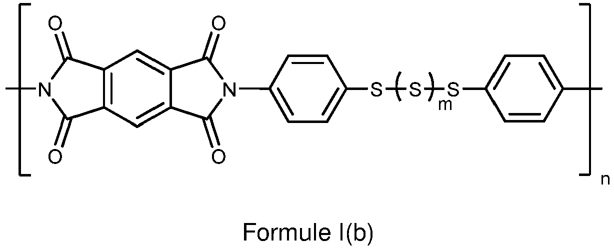 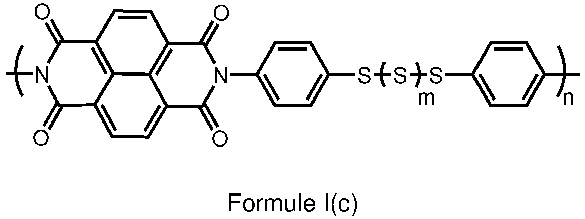 dans lesquelles m et n sont tels que définis ici.

Selon un autre exemple, le matériau actif est plutôt un composé de Formule Il : 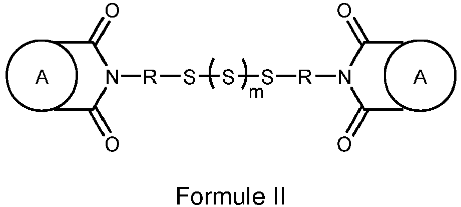 dans laquelle A et R sont tels qu'ici définis et m représente le nombre d'atomes de soufre inséré dans le pont disulfure du composé, par exemple, m > 0, par exemple 0 < m ≤ 8, ou 1 ≤ m ≤ 6, ou 1 ≤ m ≤ 4.

Par exemple, A est un benzène et le polymère est de Formule II(a) : 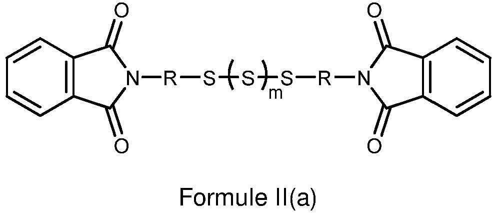 dans laquelle R et m sont tels que définis ici.

Par exemple, R est un benzène et le polymère est de Formule II(b) : 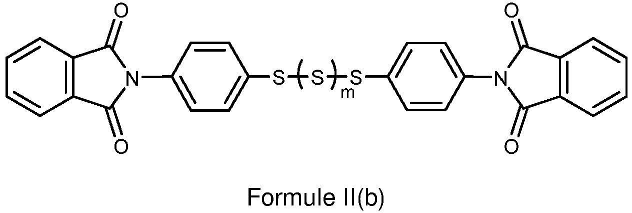 dans laquelle m est tel que défini ci-dessus.

La présente demande décrit aussi un procédé de synthèse des polymères tels qu'ici définis et comprenant un hybride organo-sulfuré comprenant des étapes de polymérisation et de sulfuration par réaction avec du soufre élémentaire, ces étapes étant effectuées dans un ordre ou l'autre. Par exemple, le procédé comprend les étapes suivantes :
a) polymérisation par polycondensation entre un dianhydride et un disulfure de diamine afin de former un polyimide électrochimiquement actif; et
b) insertion d'un segment polysulfure (de forme -(S)ₘ-; où m ≥ 1), par copolymérisation du polyimide électrochimiquement actif et du soufre élémentaire S₈.

Selon un exemple, le segment organique du copolymère est synthétisé par polycondensation entre le dianhydride pyromellitique (1) et le disulfure de 4-aminophényle (2) pour former le polyimide (3). Par exemple, la polycondensation s'effectue à une température d'environ 150 °C. Le copolymère peut être préparé par un procédé de polymérisation comme illustré dans le Schéma 1:

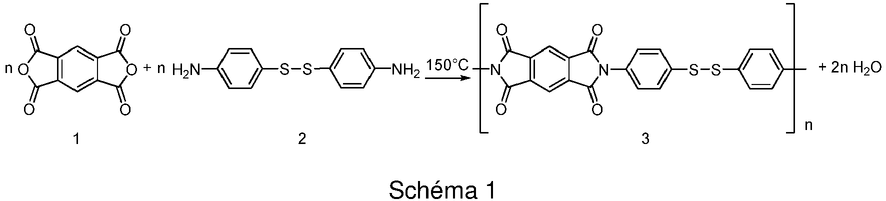

Selon un exemple, le segment polysulfure (de forme -(S)ₘ-; où m ≥ 1) est ensuite inséré au niveau du lien disulfure. L'insertion peut alors s'effectuer en chauffant à une température de 150°C ou plus, ou 185°C ou plus, ou se situant entre 160°C et 200°C, le polyimide électrochimiquement actif comprenant le pont disulfure ainsi que du soufre élémentaire S₈. L'étape de chauffage permet l'ouverture du cycle du soufre élémentaire S₈ afin d'insérer un segment polysulfure (de forme -(S)ₘ-; où m ≥ 1) dans le lien disulfure et de former un polysulfure. L'insertion du segment polysulfure peut être effectuée par un procédé tel qu'illustré dans le Schéma 2 dans lequel l'insertion du segment polysulfure (de forme -(S)ₘ-; où m ≥ 1) est effectuée sur le pont disulfure du polyimide (3) à 185°C afin d'obtenir un polyimide-co-polysulfure (4):

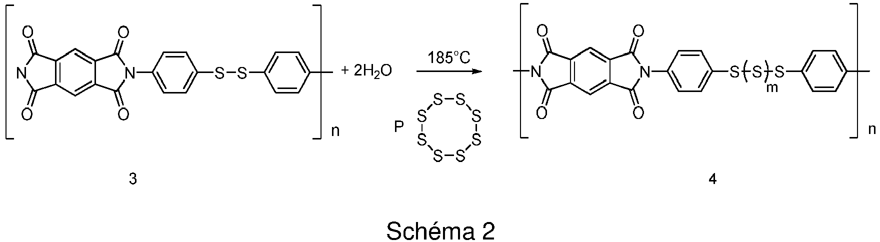

Dans l'alternative, la présente demande décrit un procédé synthèse des polymères tels qu'ici définis et comprenant un hybride organo-sulfuré comprenant les étapes suivantes :
a) synthèse d'un co-monomère amino-soufré par l'insertion d'un segment (de forme -(S)ₘ-; où m ≥ 1) formant un polysulfure s'effectuant par polymérisation d'un disulfure de diamine et de soufre élémentaire S₈; et
b) polycondensation du co-monomère amino-soufré et du dianhydride afin de former le polyimide.

Selon un exemple, le segment polysulfure (de forme -(S)ₘ-; où m ≥ 1) est inséré au niveau du lien disulfure du disulfure de diamine. L'insertion peut s'effectuer en chauffant à une température de 150°C ou plus, ou 185°C ou plus, ou se situant entre 160°C et 200°C, le soufre élémentaire S₈ et le disulfure de de diamine comprenant le pont disulfure. L'étape de chauffage permet l'ouverture du cycle du soufre élémentaire S₈ afin d'insérer un segment polysulfure dans le lien disulfure afin de former un polysulfure. L'insertion du segment polysulfure peut être effectuée par un procédé tel qu'illustré au Schéma 3 dans lequel l'insertion du segment polysulfure est effectuée à 185°C sur le pont disulfure du disulfure de 4-aminophényle (2) afin d'obtenir un co-monomère amino-soufré (3):

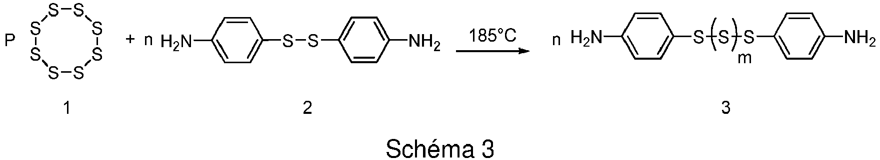

Selon un exemple, la polymérisation s'effectue ensuite par polycondensation entre le co-monomère amino-soufré (3) et le dianhydride pyromellitique (4) pour former le polyimide-co-polysulfure (5). La polycondensation peut s'effectuer, par exemple, à une température d'environ 150 °C. Le polymère peut être préparé par un procédé de polymérisation tel qu'illustré dans le Schéma 4:

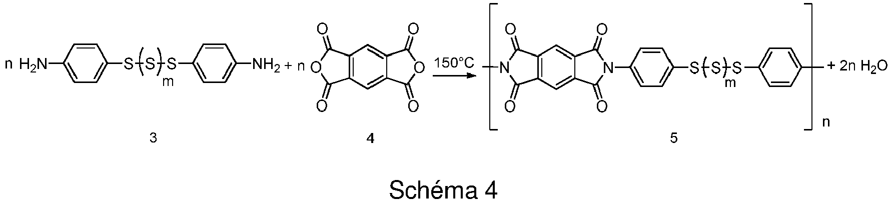

Dans lequel les valeurs de m et n sont déterminées en fonction du pourcentage massique de soufre dans le mélange réactionnel. Le pourcentage massique de soufre dans le copolymère peut varier de 0,1% à 99,9%, par exemple, entre 5% et 95%. Le polymère peut aussi contenir une certaine quantité résiduelle de soufre élémentaire libre non-inséré dans le lien disulfure, particulièrement lorsqu'une grande proportion de soufre est incluse à l'étape d'insertion.

La présente demande propose également un matériau d'électrode positive comprenant un polymère ou composé tel qu'ici défini comme matériau électrochimiquement actif. Selon un exemple, le matériau d'électrode positive peut comprendre en outre un matériau conducteur d'électrons, un liant, ou une combinaison. Le matériau d'électrode peut aussi contenir du soufre élémentaire libre (Sₓ).

Des exemples non limitatifs de matériau conducteur peuvent comprendre une source de carbone telle que le noir de carbone, le carbone Ketjen^{™}, le carbone Shawinigan, le noir d'acétylène, le graphite, le graphène, les fibres de carbone (telles les nanofibres de carbone (par exemple, VGCF formé en phase gazeuse)), et les nanotubes de carbone, ou une combinaison d'au moins deux de ceux-ci. Par exemple, le matériau conducteur est une combinaison de VGCF et de Ketjen^{™} Black.

Des exemples non limitatifs de liants comprennent un polymère de type polyéther linéaire, ramifié et/ou réticulé et pouvant être basé sur le poly(oxyde d'éthylène) (PEO), le poly(oxyde de propylène) (PPO) ou d'un mélange des deux (ou un co-polymère EO/PO), et comprenant éventuellement des unités réticulables; un polymère fluoré tel que le fluorure de polyvinylidène (PVDF) ou le polytétrafluoroéthylène (PTFE); ou un liant soluble dans l'eau tel que le caoutchouc styrène-butadiène (SBR), le caoutchouc acrylonitrile-butadiène (NBR), le NBR hydrogéné (HNBR), le caoutchouc d'épichlorohydrine (CHR), ou caoutchouc d'acrylate (ACM), et comprenant optionnellement du carboxyméthylcellulose (CMC). Par exemple, le liant est le PVDF et est dissout dans le N-méthyl-2-pyrrolidone (NMP) lorsque mélangé aux autres composantes d'un matériau d'électrode positive pour l'épandage, le solvant étant ensuite éliminé.

Selon un exemple, le matériau d'électrode positive peut être appliqué sur un collecteur de courant (par exemple, aluminium, cuivre) alternativement l'électrode positive peut être autosupportée. Par exemple, le collecteur de courant est l'aluminium.

La présente demande propose également une cellule électrochimique comprenant au moins le matériau d'électrode positive tel qu'ici défini, une électrode négative et un électrolyte. L'électrolyte est alors choisi pour sa compatibilité avec les différents éléments de la batterie. Tout type électrolyte est envisagé, par exemple, les électrolytes liquides, gels ou solides.

La présente demande propose également un électrolyte comprenant un polymère tel qu'ici défini ou un composé tel qu'ici défini.

Selon un exemple, l'électrolyte est un électrolyte liquide comprenant un sel dans un solvant. Selon une alternative, l'électrolyte est un électrolyte en gel comprenant un sel dans un solvant et optionnellement un polymère solvatant. Selon une autre alternative, l'électrolyte est un électrolyte polymère solide (SPE) comprenant un sel dans un polymère solvatant.

Les électrolytes compatibles comprennent généralement au moins un sel de lithium tel que l'hexafluorophosphate de lithium (LiPF₆), le bis(trifluoromethanesulfonyl)imidure de lithium (LiTFSI), le bis(fluorosulfonyl)imidure de lithium (LiFSI), le 2-trifluorométhyl-4,5-dicyano-imidazolate de lithium (LiTDI), le 4,5-dicyano-1,2,3-triazolate de lithium (LiDCTA), le bis (pentafluoroéthylsulfonyl) imide de lithium (LiBETI), le tétrafluoroborate de lithium (LiBF₄), le bis(oxalato) borate de lithium (LiBOB), le nitrate de lithium (LiN0₃), le chlorure de lithium (LiCI), le bromure de lithium (LiBr), le fluorure de lithium (LiF), et les compositions les comprenant dissous dans un solvant non aqueux (organique) ou dans un polymère solvatant. Par exemple, l'électrolyte est le LiTFSI dissout dans le diméthoxyéthane (DME) et de 1,3-dioxolane (DOL) (DME/DOL) et de nitrate de lithium (1%, LiNOs).

Selon un autre exemple, l'électrolyte peut comprendre en outre du soufre élémentaire, un liant d'électrolyte, un séparateur, un additif ou une combinaison d'au moins deux de ceux-ci.

Par exemple, le liant d'électrolyte est le PVDF (fluorure de polyvinylidène). Par exemple, l'électrolyte comprend entre 0 % en masse et 25 % en masse, particulièrement entre 0 % en masse et 20 % en masse, plus particulièrement entre 5 % en masse et 15 % en masse, encore plus particulièrement entre 7 % en masse et 13 % en masse et idéalement 10 % en masse de liant d'électrolyte, bornes supérieures et inférieures incluses.

Des exemples non limitatifs de séparateur peuvent comprendre des membranes de polyéthylène (PE), de polypropylène (PP), de cellulose, de polytétrafluoroéthylène (PTFE), poly(fluorure de vinylidène) (PVDF) et de polypropylène-polyéthylène-polypropylène (PP/PE/PE).

Selon un autre exemple, l'électrolyte est un électrolyte polymère solide (SPE) comprenant un sel tel qu'ici défini dans un polymère solvatant dans lequel le polymère solvatant est le polymère tel qu'ici défini. Par exemple, le SPE comprend entre 60 % en masse et 95 % en masse, particulièrement entre 65 % en masse et 90 % en masse, plus particulièrement entre 70 % en masse et 85 % en masse, encore plus particulièrement entre 75 % en masse et 85 % en masse et idéalement 80 % en masse du polymère tel qu'ici défini, bornes supérieures et inférieures incluses. Par exemple, le SPE comprend entre 5 % en masse et 40 % en masse, particulièrement entre 10 % en masse et 35 % en masse, plus particulièrement entre 15 % en masse et 30 % en masse, encore plus particulièrement entre 15 % en masse et 25 % en masse et idéalement 20 % en masse de sel, bornes supérieures et inférieures incluses.

La présente demande propose également une cellule électrochimique comprenant une cathode, une anode et un électrolyte tel qu'ici défini. Par extension, la présente demande envisage aussi une cellule électrochimique comprenant une cathode, une anode et un électrolyte, dans laquelle l'électrolyte et la cathode sont tels qu'ici définis.

Selon un autre aspect, une cellule électrochimique de la présente demande est comprise dans un accumulateur au lithium. Par exemple, l'accumulateur au lithium est une batterie lithium-soufre.

Selon un autre aspect, les cellules électrochimiques de la présente demande sont utilisées dans des appareils nomades, par exemple les téléphones portables, les appareils photo, les tablettes ou les ordinateurs portables, dans des véhicules électriques ou hybrides, ou dans le stockage d'énergie renouvelable.

### EXEMPLES

Les exemples qui suivent sont à titre d'illustration et ne doivent pas être interprétés comme limitant davantage la portée de l'invention telle que décrite.

### Exemple 1 - Synthèse de matériaux actifs d'électrodes hybrides organosulfurés

### a) Copolymérisation de dianhydride pyromellitique, de disulfure de 4-aminophényle et de soufre (5% en masse)

La synthèse du matériau d'électrode a été effectuée dans un flacon de 25 mL muni d'un barreau magnétique. Le matériau actif d'électrode a été préparé en combinant 0,085 g de soufre élémentaire S₈ (0,33 mmol) et 0,745 g de disulfure de 4-aminophényle (3 mmol). Le flacon a ensuite été fermé hermétiquement et mis sous un flux de gaz inerte (N₂). Une fois l'atmosphère du flacon purgé, le mélange a été chauffé à une température de 185°C sous agitation constante de 500 rpm durant environ 30 minutes, jusqu'à l'obtention d'un liquide homogène. Le mélange a ensuite été refroidi à une température de 150°C et le bouchon hermétique a ensuite été retiré afin d'ajouter 0,654 g de dianhydride pyromellitique (3 mmol) préalablement dissout dans un solvant organique, le N,N-diméthylformamide (DMF). Le flacon contenant le mélange a ensuite été recouvert et gardé à une température de 150°C sous agitation (500 rpm) pour une durée de 26 heures. Le mélange a ensuite été refroidi. Le solide ainsi produit a été récupéré, lavé avec du tétrahydrofurane (THF) et séché sous vide afin de sublimer le soufre n'ayant pas réagi. Finalement, le pourcentage de soufre dans le matériau actif d'électrode (polymère) produit a été déterminé par analyse élémentaire. Le matériau actif d'électrode du présent exemple comprenait 5% de soufre.

### b) Copolymérisation de dianhydride pyromellitique, de disulfure de 4-aminophényle et de soufre (25% en masse)

La synthèse d'un matériau actif d'électrode a été effectuée selon le procédé présenté à l'Exemple 1(a). Les masses de soufre élémentaire S₈ et de disulfure de 4-aminophényle pour la synthèse du co-monomère amino-soufré étaient respectivement de 0,309 g (1,206 mmol) et de 0,447 g (1,8 mmol) et la masse de dianhydride pyromellitique pour la polycondensation était de 0,393 g (1,8mmol). Le matériau actif d'électrode ainsi produit comprenait 25% en masse de soufre tel que déterminé par analyse élémentaire.

### c) Copolymérisation de dianhydride pyromellitique, de disulfure de 4-aminophényle et de soufre (35% en masse)

La synthèse d'un matériau actif d'électrode a été effectuée selon le procédé présenté à l'Exemple 1(a). Les masses de soufre élémentaire S₈ et de disulfure de 4-aminophényle pour la synthèse du co-monomère amino-soufré étaient respectivement de 1,033 g (4,026 mmol) et de 1 g (4,026 mmol) et la masse de dianhydride pyromellitique pour la polycondensation était de 0,878 g (4,026 mmol). Le matériau actif d'électrode ainsi produit comprenait 35% en masse de soufre tel que déterminé par analyse élémentaire.

### d) Copolymérisation de dianhydride pyromellitique, de disulfure de 4-aminophényle et de soufre (63% en masse)

La synthèse de ce matériau actif d'électrode a été effectuée selon le procédé présenté à l'Exemple 1(a). Les masses de soufre élémentaire S₈ et de disulfure de 4-aminophényle pour la synthèse du co-monomère amino-soufré étaient respectivement de 2,463 g (9,6 mmol) et de 0,397 g (1,6 mmol) et la masse de dianhydride pyromellitique pour la polycondensation était de 0,349 g (1,6 mmol). Le matériau actif d'électrode ainsi obtenu comprenait 63% en masse de soufre tel que déterminé par analyse élémentaire.

### e) Copolymérisation de dianhydride pyromellitique, de disulfure de 4-aminophényle et de soufre (95% en masse)

La synthèse de ce matériau actif d'électrode a été effectuée selon le procédé présenté à l'Exemple 1 (a). Les masses de soufre élémentaire S₈ et de disulfure de 4-aminophényle pour la synthèse du co-monomère amino-soufré étaient respectivement de 8,978 g (35 mmol) et de 0,248 g (1 mmol) et la masse de dianhydride pyromellitique pour la polycondensation était de 0,218 g (1 mmol). Le matériau actif d'électrode ainsi produit comprenait 95% en masse de soufre tel que déterminé par analyse élémentaire.

### f) Copolymérisation de dianhydride de naphtalène-1,4,5,8-tétracarboxylique, de disulfure de 4-aminophényle et de soufre (20 % en masse)

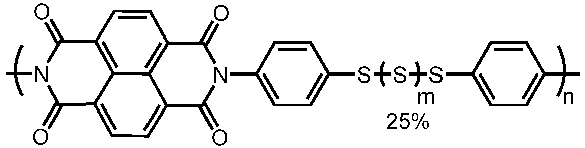

La synthèse du matériau d'électrode a été effectuée dans un flacon de 25 mL muni d'un barreau magnétique. Le matériau actif d'électrode a été préparé en combinant 0,923 g de soufre élémentaire S₈ (3,6 mmol) et 0,447 g de disulfure de 4-aminophényle (1,8 mmol). Le flacon a ensuite été fermé hermétiquement et mis sous un flux de gaz inerte (N₂). Une fois l'atmosphère du flacon purgé, le mélange a été chauffé à une température de 185°C sous agitation constante de 500 rpm durant environ 30 minutes, jusqu'à l'obtention d'un liquide homogène. Le mélange a ensuite été refroidi à une température de 150°C et le bouchon hermétique a ensuite été retiré afin d'ajouter 0,483 g de dianhydride de naphtalène-1,4,5,8-tétracarboxylique (1,8 mmol) préalablement dissout dans un solvant organique, le N,N-diméthylformamide (DMF). Le flacon contenant le mélange a ensuite été recouvert et gardé à une température de 150°C sous agitation (500 rpm) pour une durée de 26 heures. Le mélange a ensuite été refroidi. Le solide ainsi produit a été récupéré, lavé avec du tétrahydrofurane (THF) et séché sous vide afin de sublimer le soufre n'ayant pas réagi. Finalement, le pourcentage de soufre dans le matériau actif d'électrode (polymère) produit a été déterminé par analyse élémentaire. Le matériau actif d'électrode du présent exemple comprenait 20% de soufre.

### g) Copolymérisation de dianhydride de naphtalène-1,4,5,8-tétracarboxylique, de disulfure de 4-aminophényle et de soufre (30 % en masse)

La synthèse d'un matériau actif d'électrode a été effectuée selon le procédé présenté à l'Exemple 1(f). Les masses de soufre élémentaire S₈ et de disulfure de 4-aminophényle pour la synthèse du co-monomère amino-soufré étaient respectivement de 2,052 g (8 mmol) et de 1,987 g (8 mmol) et la masse de dianhydride de naphtalène-1,4,5,8-tétracarboxyliquepour la polycondensation était de 2,145 g (8 mmol). Le matériau actif d'électrode ainsi produit comprenait 30% en masse de soufre tel que déterminé par analyse élémentaire.

### h) Copolymérisation de dianhydride pyromellitique et de cystamine par polycondensation suivie de l'insertion du soufre (20 % en masse)

La synthèse du copolymère a été effectuée dans un flacon de 100 mL muni d'un barreau magnétique. Le matériau a été préparé en combinant 7,614 g de dichlorhydrate de cystamine (0,5 mol) et 10,91 g de dianhydride pyromellitique (0,5 mol). Le mélange a ensuite été chauffé à une température de 150°C sous agitation (500 rpm) pour une durée de 26 heures. Le mélange a ensuite été refroidi. Le solide ainsi produit a été récupéré, lavé avec du tétrahydrofurane (THF). 1,204 g de ce copolymère a ensuite été dispersé dans du NMP puis 1,846g de soufre élémentaire S₈ (50% en masse) a été ajouté. Le flacon a ensuite été fermé hermétiquement et mis sous un flux de gaz inerte (N₂). Une fois l'atmosphère du flacon purgé, le mélange a été chauffé à une température de 185°C sous agitation constante de 500 rpm durant environ 20 heures. Le mélange a ensuite été refroidi. Le solide ainsi produit a été récupéré, lavé avec du tétrahydrofurane (THF) et séché sous vide afin de sublimer le soufre n'ayant pas réagi. Finalement, le pourcentage de soufre dans le matériau actif d'électrode (polymère) produit a été déterminé par analyse élémentaire. Le matériau actif d'électrode du présent exemple comprenait 20 % de soufre.

### Exemple 2 - Préparation de cellules électrochimiques

### a) Cathode

Le matériau de la cathode est composé de 60% en masse du matériau d'électrode organo-sulfuré hybride, soit le polyimide-co-polysulfure de l'Exemple 1, soit de polyimide disulfure (référence), 30% en masse de carbone conducteur (15% VGCF et 15% de carbone Ketjen^{MC} 600) et de 10% en masse de liant, soit le poly(fluorure de vinylidène) (PVDF) dissout dans le N-méthyl-2-pyrrolidone (NMP). Le collecteur de courant est l'aluminium. Le solvant est évaporé après l'épandage.

### b) Électrolyte

L'électrolyte consiste en une solution de concentration 1M de bis(trifluoromethanesulfonyl)imide de lithium (LiTFSI) dissout dans un mélange de diméthoxyéthane et de 1,3-dioxolane (DME/DOL) et de nitrate de lithium (1%, LiNOs).

### c) Anode

L'anode est composée de lithium métallique sous forme de film mince.

### d) Cellule électrochimique

Des cellules sont donc préparées avec les éléments suivants :
Li / Électrolyte / Cathode / Al

### Cellule 1

La Cellule 1 comprend la cathode référence comprenant du polyimide disulfure présenté à l'Exemple 2 (a), l'électrolyte présenté à l'Exemple 2 (b) et une anode composée de lithium métallique présenté à l'Exemple 2 (c).

### Cellule 2

La Cellule 2 comprend la cathode présentée à l'Exemple 2 (a) qui comprend le matériau d'électrode de l'Exemple 1(c), l'électrolyte présenté à l'Exemple 2 (b) et une anode composée de lithium métallique présenté à l'Exemple 2 (c).

### Cellule 3

La Cellule 3 comprend la cathode présentée à l'Exemple 2 (a) qui comprend le matériau d'électrode de l'Exemple 1 (d), l'électrolyte présenté à l'Exemple 2 (b) et une anode composée de lithium métallique présenté à l'Exemple 2 (c).

### Cellule 4

La Cellule 4 comprend la cathode présentée à l'Exemple 2 (a) qui comprend le matériau d'électrode de l'Exemple 1(e), l'électrolyte présenté à l'Exemple 2 (b) et une anode composée de lithium métallique présenté à l'Exemple 2 (c).

### Cellule 5

La Cellule 5 comprend la cathode présentée à l'Exemple 2 (a) qui comprend le matériau d'électrode de l'Exemple 1(b), l'électrolyte présenté à l'Exemple 2 (b) et une anode composée de lithium métallique présenté à l'Exemple 2 (c).

### Cellule 6

La Cellule 6 comprend la cathode présentée à l'Exemple 2 (a) qui comprend le matériau d'électrode de l'Exemple 1 (f), l'électrolyte présenté à l'Exemple 2 (b) et une anode composée de lithium métallique présenté à l'Exemple 2 (c).

### Cellule 7

La Cellule 7 comprend la cathode présentée à l'Exemple 2 (a) qui comprend le matériau d'électrode de l'Exemple 1 (g), l'électrolyte présenté à l'Exemple 2 (b) et une anode composée de lithium métallique présenté à l'Exemple 2 (c).

### Cellule 8

La Cellule 8 comprend la cathode présentée à l'Exemple 2 (a) qui comprend le matériau d'électrode de l'Exemple 1 (h) (sans ajout de soufre), l'électrolyte présenté à l'Exemple 2 (b) et une anode composée de lithium métallique présenté à l'Exemple 2 (c).

### Cellule 9

La Cellule 9 comprend la cathode présentée à l'Exemple 2 (a) qui comprend le matériau d'électrode de l'Exemple 1 (a) contenant 20% en masse de soufre, l'électrolyte présenté à l'Exemple 2 (b) et une anode composée de lithium métallique présenté à l'Exemple 2 (c).

### Exemple 3 - Propriétés électrochimiques

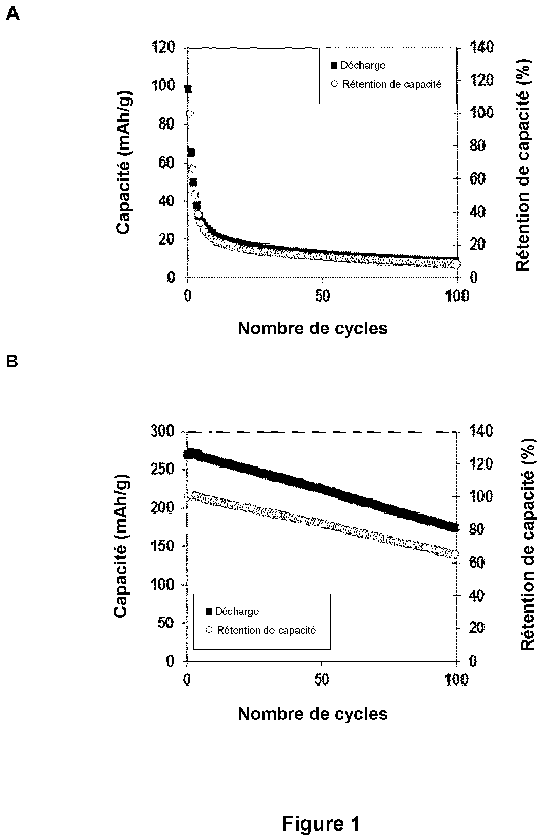

Les mesures électrochimiques ont été effectuées sur les cellules électrochimiques de l'Exemple 2(d). Comme démontré avec la Figure 1, la présence de soufre inséré dans le pont disulfure permet l'obtention d'un matériau plus performant ayant une capacité et une cyclabilité améliorées. Les Figures 1 (A) et 1(B) présentent respectivement les résultats de capacité et de rétention de la capacité en fonction du nombre de cycles pour la Cellule 1 (référence) et pour la Cellule 2. En effet, en comparant les résultats présentés, une amélioration significative de la capacité ainsi que du pourcentage de rétention de la capacité est observable pour la Cellule 2 en comparaison de la Cellule 1.

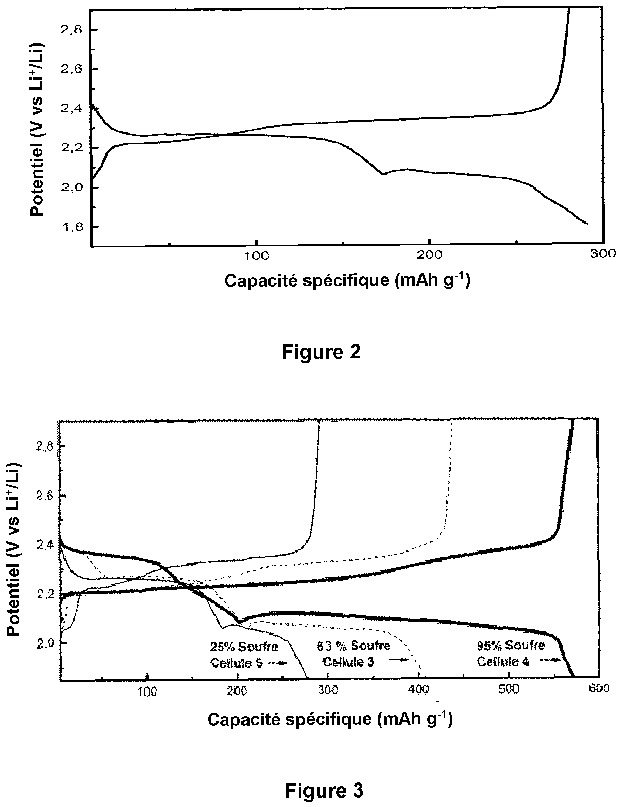

La Figure 2 présente les résultats de capacité en fonction du voltage pour le premier cycle en C/10 de la Cellule 2 dans lequel la contribution du polymère à la capacité du nouveau matériau peut être constatée par la présence du plateau à environ 2,2V. La Cellule 2 obtient une capacité de première décharge de 290 mAhg⁻¹ en C/10 et un pourcentage de rétention de capacité de 65% après 100 cycles en C/2.

La Figure 3 présente la première courbe de charge-décharge des Cellules 3 à 5 en C/10, où la capacité initiale varie en fonction de la nature du matériau actif. La capacité obtenue avec la Cellule 3, la Cellule 4 et la Cellule 5 est respectivement de 407 mAhg⁻¹, de 570 mAhg⁻¹ et de 277 mAhg⁻¹.

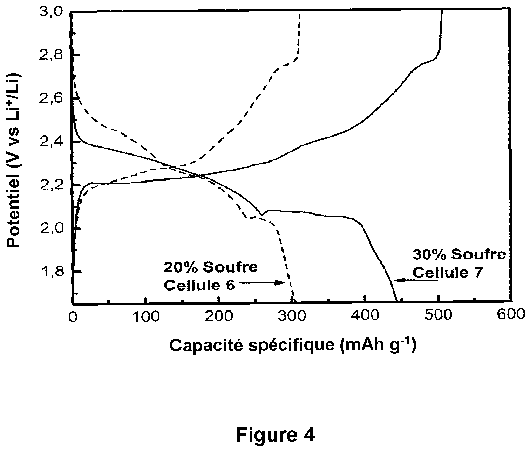

La Figure 4 présente les résultats de capacité en fonction du voltage pour le premier cycle en C/10 des Cellules 6 et 7 dans lequel la contribution du soufre à la capacité du nouveau matériau peut être constatée par la présence du plateau à environ 2,05V. La Cellule 6 obtient une capacité de première décharge de 305 mAhg⁻¹ en C/10 et un pourcentage de rétention de capacité de 91% après 50 cycles en C/2. Pour la Cellule 7, la capacité de la première décharge est de 443 mAhg⁻¹ en C/10 et présente un pourcentage de rétention de capacité de 75% après 50 cycles.

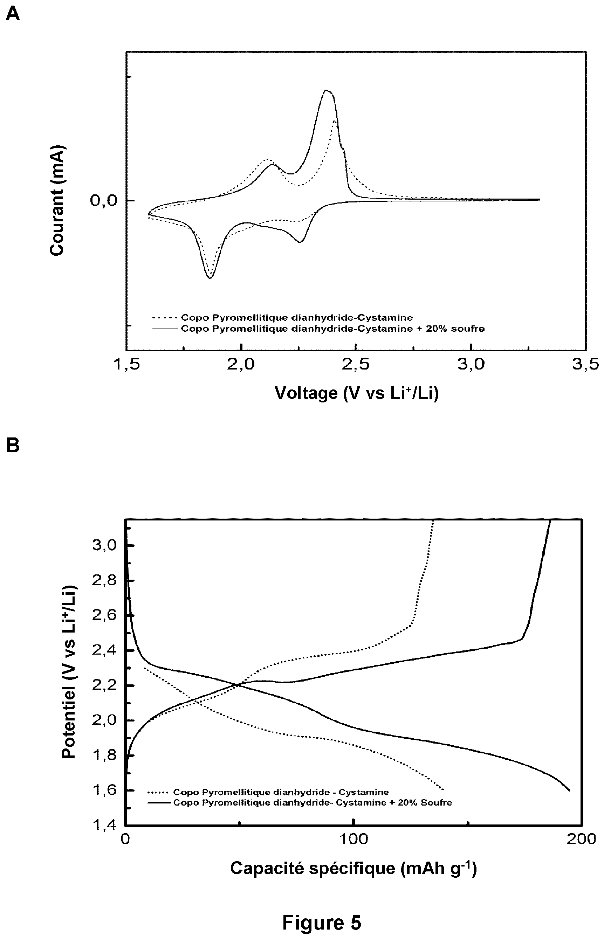

La Figure 5 (a) présente les cyclovoltamogrammes des Cellules 8 et 9. Il est possible de constater l'effet de l'ajout de soufre sur l'intensité des pics de réduction et d'oxydation (redox). De plus, on obtient une augmentation de la capacité lors de l'ajout de soufre observable à la Figure 5 (b). La Cellule 8 obtient une capacité de première décharge de 138 mAhg⁻¹ en C/10 et un pourcentage de rétention de capacité de 37% après 100 cycles en C/2. Pour la Cellule 9, la capacité obtenue à la première décharge est de 195 mAhg⁻¹ en C/10 et présente un pourcentage de rétention de capacité de 50% après 100 cycles

### Exemple 4 - Caractérisation et détermination de la composition des matériaux

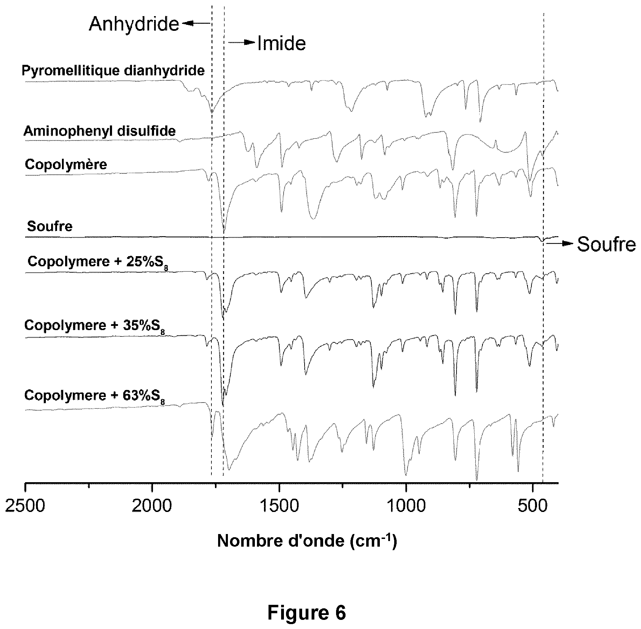

La composition des matériaux présentés à l'Exemple 1 a été déterminée à l'aide de spectres de transmission infrarouge. La Figure 6 présente les spectres de transmission infrarouge des matériaux actifs d'électrode présentés aux Exemples 1 (b), (c), (d) ainsi que les spectres des monomères utilisés lors de la copolymérisation, du copolymère imide sans ajout de soufre et le spectre du soufre élémentaire.

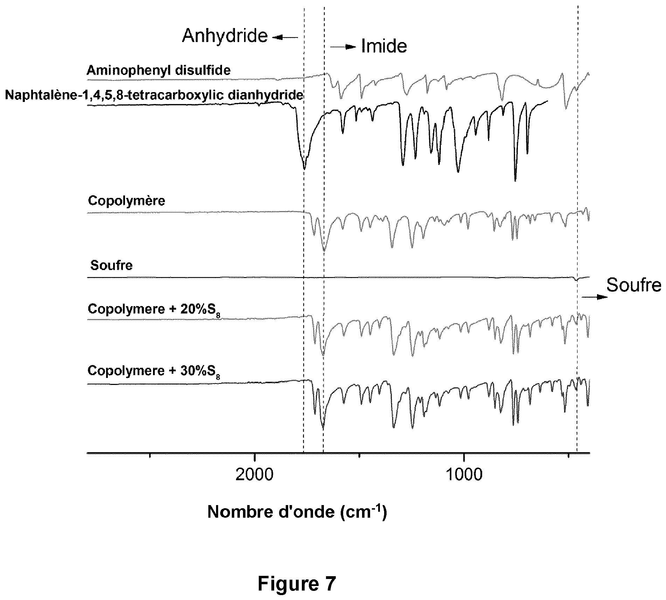

La Figure 7 présente les spectres de transmission infrarouge des matériaux actifs d'électrode présentés aux Exemples 1(f) et 1(g) ainsi que les spectres des monomères utilisés lors de la copolymérisation, du soufre élémentaire et du copolymère sans soufre ajouté.

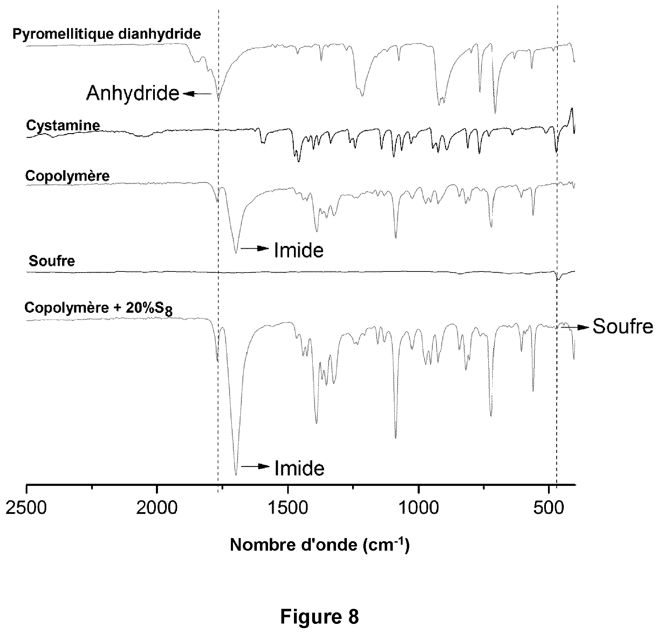

La Figure 8 présente les spectres de transmission infrarouge du matériau actif d'électrode présenté à l'Exemple 1(h) ainsi que les spectres des monomères utilisés lors de la copolymérisation, du soufre élémentaire et du copolymère sans soufre ajouté.

### Exemple 5 - Conductivités ioniques

### a) Mesure de la conductivité ionique du matériau présenté à l'Exemple 1(c)

Les résultats de conductivité ionique ont été obtenus pour le copolymère présenté à l'Exemple 1(c). Pour ce faire, le copolymère présenté à l'Exemple 1(c) a été mélangé dans un solvant organique (NMP) avec un sel de lithium (LiTFSI) et avec un liant (PVdF). La dispersion homogène a été épandue sur une feuille d'acier inoxydable d'une épaisseur de 27 µm. Le film a ensuite été séché 16 heures à une température de 120°C. Le NMP étant évaporé, un ratio (copolymère : LiTFSI : PVdF) de 70 : 20 : 10 (pourcentage en masse) a été obtenu.

Le film ainsi obtenu a ensuite été placé entre deux électrodes d'acier inoxydable et assemblé en pile bouton afin de mesurer la conductivité ionique. La spectroscopie d'impédance électrochimique a été effectuée entre 800 kHz et 100 Hz à différentes températures (25°C, 40°C, 60°C et 80°C).

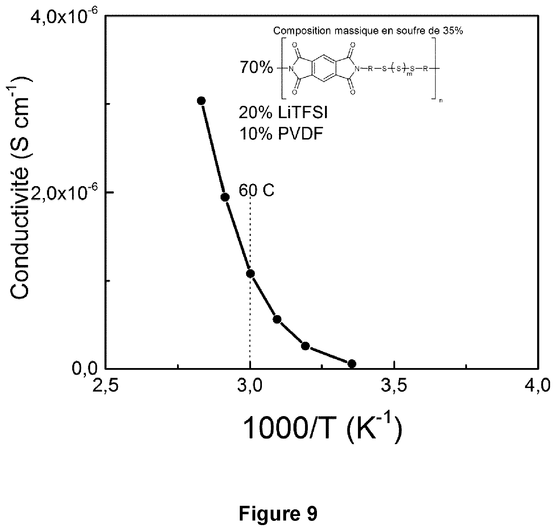

Le graphique, à la Figure 9, présente les valeurs de conductivité mesurées en fonction de la température. Une valeur maximale de 3.06 × 10⁻⁶ S.cm⁻¹ a été obtenue à une température de 80°C.

### b) Mesure de la conductivité ionique du matériau présenté à l'Exemple 1(d)

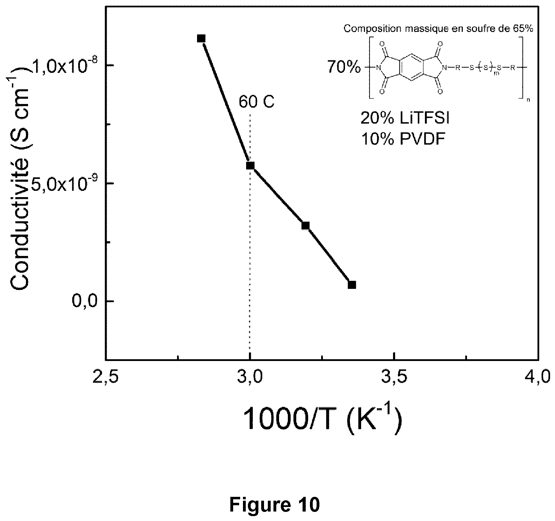

La conductivité en fonction de la température du copolymère soufré présenté à l'Exemple 1(d), a été mesurée en utilisant la méthode et les ratios présentés à l'Exemple 5(a). Les résultats de conductivité ionique sont présentés à la Figure 10. Une valeur maximale de 1.12 × 10⁻⁸ S.cm⁻¹ a été obtenue à une température de 80 °C.

### c) Mesure de la conductivité ionique du matériau présenté à l'Exemple 1(g)

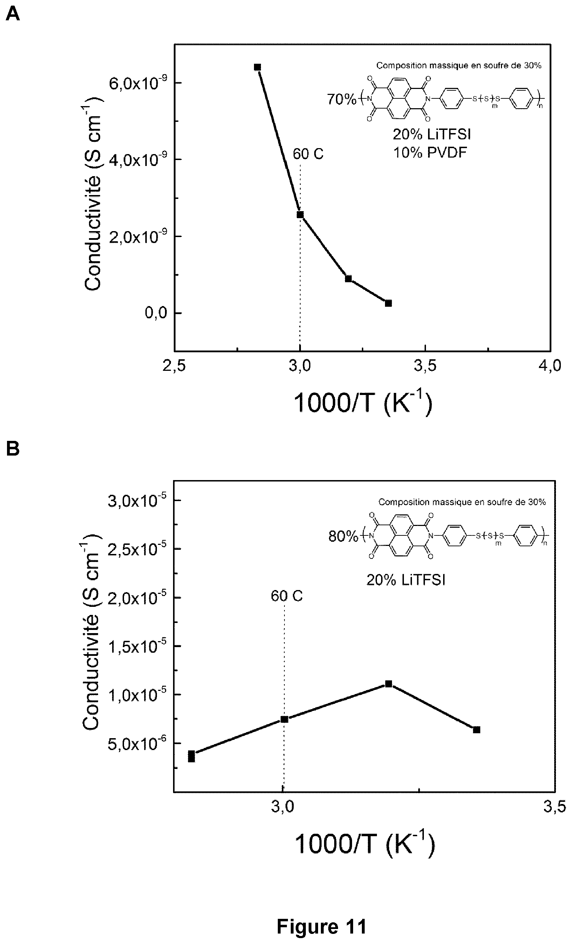

La conductivité en fonction de la température du copolymère soufré présenté à l'Exemple 1(g) a été mesurée en utilisant la méthode et les ratios présentés à l'Exemple 5(a). Les résultats sont présentés à la figure 11(A). Une valeur maximale de 6.43 × 10-⁹ S.cm⁻¹ a été obtenue à une température de 80°C.

### d) Mesure de la conductivité ionique du matériau présenté à l'Exemple 1(g)

La conductivité ionique a été mesurée une seconde fois pour le copolymère soufré présenté à l'Exemple 1(g). Pour ce faire, le copolymère soufré présenté à l'Exemple 1(g) a été mélangé avec 20 % en masse d'un sel de lithium (LiTFSI). Ce mélange de solide a été pressé, entre deux électrodes d'acier inoxydable, à une température de 150°C pour une durée de 30 minutes. La spectroscopie d'impédance électrochimique a ensuite été mesurée entre 800 kHz et 100 Hz à différentes températures (25°C, 40°C, 60°C et 80°C). Les résultats sont présentés à la Figure 11(B). Une conductivité ionique maximale de 1.1 ×10⁻⁵ S.cm⁻¹ a été obtenue à une température de 40°C.

## Revendications

1. Polymère de Formule I: 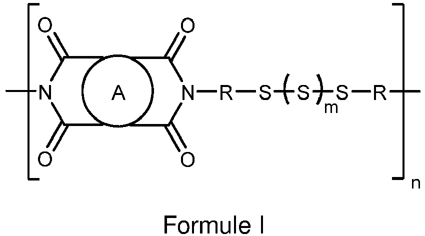 dans laquelle :
A est choisi parmi les groupements insaturés permettant la délocalisation des électrons, par exemple, les groupements aryles et hétéroaryles substitués ou non substitués, et leurs équivalents polycycliques fusionnés ou non, de préférence A étant choisi parmi les groupements benzène, naphtalène, pérylène, et biphényle;
R est un groupement organique de liaison substitué ou non substitué choisi parmi C₂₋₆alkylène linéaire ou ramifié, C₂₋₆alkylèneoxy linéaire ou ramifié, C₂₋₆alkylèneglycol linéaire ou ramifié, C₂₋₆alkylèneoxyC₂₋₆alkylène linéaire ou ramifié, poly(C₂₋₆alkylèneglycol) linéaire ou ramifié, C₆₋₁₂arylène, C₃₋₁₂cycloalkylène, C₅₋₁₂hétéroarylène, C₃₋₁₂hétérocycloalkylène, de préférence R étant choisi parmi les groupements benzène, éthylène, propylène, poly(éthylène glycol), poly(propylène glycol), et les copolymères d'éthylène glycol et de propylène glycol;
m représente le nombre moyen d'atomes de soufre insérés dans le pont disulfure des maillons du polymère et ne peut être nul, c'est-à-dire m > 0, par exemple 0 < m ≤ 8, ou 1 ≤ m ≤ 6, ou 1 ≤ m ≤ 4; et
n représente le nombre moyen d'unités dans le polymère, par exemple, n peut être situé dans l'intervalle de 2 à 500, ou de 5 à 300.

2. Polymère selon la revendication 1, le polymère étant de Formule I(a) : 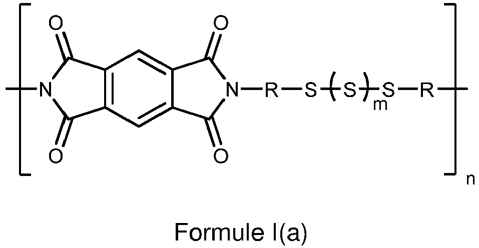 dans laquelle R, m et n sont tels que définis à la revendication 1.

3. Polymère selon la revendication 2, le polymère étant de Formule I(b): 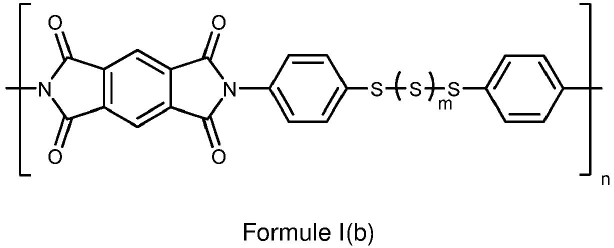 dans laquelle m et n sont tels que définis à la revendication 1.

4. Composé de Formule Il : 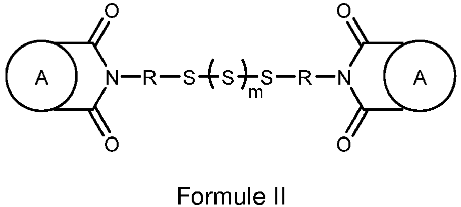 dans laquelle A et R sont tels que définis à la revendication 1, et m représente le nombre d'atomes de soufre insérés dans le pont disulfure du composé, par exemple, m > 0, par exemple 0 < m ≤ 8, ou 1 ≤ m ≤ 6, ou 1 ≤ m ≤ 4.

5. Composé selon la revendication 4, le composé étant de Formule II(a): 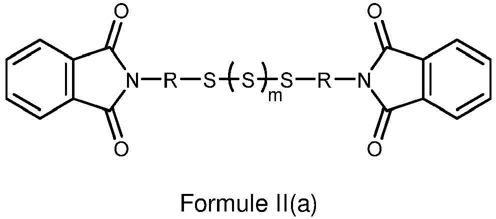 dans laquelle R et m sont tels que définis à la revendication 4.

6. Composé selon la revendication 4, le composé étant de Formule II(b): 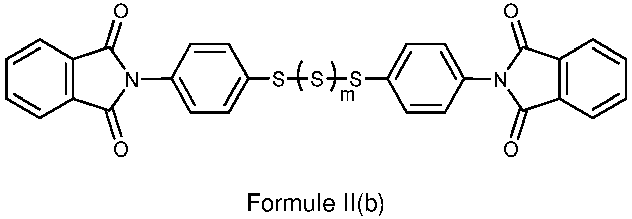 dans laquelle m est tel que défini à la revendication 4.

7. Matériau d'électrode comprenant un polymère tel que défini selon l'une quelconque des revendications 1 à 3 ou un composé tel que défini selon l'une quelconque des revendications 4 à 6, comprenant éventuellement du soufre élémentaire et/ou un matériau conducteur, un liant, ou une combinaison des deux.

8. Matériau d'électrode selon la revendication 7, dans lequel le matériau conducteur est choisi parmi le noir de carbone, le carbone Ketjen^{MC}, le carbone Shawinigan, le noir d'acétylène, le graphite, le graphène, les fibres de carbone (telles les nanofibres de carbone (par exemple, VGCF formé en phase gazeuse)), et les nanotubes de carbone, ou une combinaison d'au moins deux de ceux-ci.

9. Matériau d'électrode selon la revendication 7 ou 8, dans lequel le liant est un liant polymère de type polyéther, polymère fluoré, ou un liant soluble dans l'eau.

10. Matériau d'électrode selon la revendication 9, dans lequel :
- le liant polymère de type polyéther est linéaire, ramifié et/ou réticulé et est basé sur le poly(oxyde d'éthylène) (PEO), le poly(oxyde de propylène) (PPO) ou d'un mélange des deux (ou un co-polymère EO/PO), et comprend éventuellement des unités réticulables ; ou
- le liant polymère fluoré est le PVDF (fluorure de polyvinylidène) ou le PTFE (polytétrafluoroéthylène) ; ou
- le liant soluble dans l'eau est le SBR (caoutchouc styrène-butadiène), le NBR (caoutchouc acrylonitrile-butadiène), le HNBR (NBR hydrogéné), le CHR (caoutchouc d'épichlorohydrine), ou ACM (caoutchouc d'acrylate), comprenant optionnellement du CMC (carboxyméthylcellulose).

11. Électrode positive comprenant un matériau d'électrode selon l'une quelconque des revendications 7 à 10 appliqué sur un collecteur de courant.

12. Électrolyte comprenant un polymère tel que défini à l'une quelconque des revendications 1 à 3 ou un composé tel que défini à l'une quelconque des revendications 4 à 6, et éventuellement du soufre élémentaire.

13. Électrolyte selon la revendication 12, l'électrolyte étant un électrolyte liquide comprenant un sel dans un solvant ou un électrolyte en gel comprenant un sel dans un solvant et optionnellement un polymère solvatant.

14. Électrolyte selon la revendication 13, le solvant étant un solvant polaire aprotique choisi parmi le carbonate d'éthylène (EC), le carbonate de diéthyle (DEC), le carbonate de propylène (PC), le carbonate de diméthyle (DMC), le carbonate de méthyle et d'éthyle (EMC), la y-butyrolactone (y-BL), le carbonate de vinylène (VC), le butyrate de méthyle (MB), la y-valérolactone (y-VL), le 1,2-diméthoxyéthane (DME), le 1,2-diéthoxyéthane (DEE), le 2-méthyltétrahydrofurane, le diméthylsulfoxyde, le formamide, l'acétamide, le diméthylformamide, le dioxolane, l'acétonitrile , le propylnitrile, le nitrométhane, l'éthylmonoglyme, le triméthoxyméthane, les dérivés de dioxolane, le sulfolane, le méthylsulfolane, les dérivés de carbonate de propylène, le tétrahydrofurane et leurs mélanges.

15. Électrolyte selon la revendication 12, l'électrolyte étant un électrolyte polymère solide (SPE) comprenant un sel dans un polymère solvatant.

16. Électrolyte selon l'une quelconque des revendications 12 à 15, le polymère ou le composé étant un additif.

17. Électrolyte selon la revendication 15, le polymère solvatant étant le polymère tel que défini à l'une quelconque des revendications 1 à 3 ou le composé tel que défini à l'une quelconque des revendications 4 à 6.

18. Électrolyte selon l'une quelconque des revendications 13 à 17, dans lequel le sel est un sel de lithium, de préférence choisi parmi l'hexafluorophosphate de lithium (LiPF₆), le bis(trifluorométhanesulfonyl)imidure de lithium (LiTFSI), le bis(fluorosulfonyl)imidure de lithium (LiFSI), le 2-trifluorométhyl-4,5-dicyano-imidazolate de lithium (LiTDI), le 4,5-dicyano-1,2,3-triazolate de lithium (LiDCTA), le bis (pentafluoroéthylsulfonyl) imide de lithium (LiBETI), le tétrafluoroborate de lithium (LiBF₄), le bis(oxalato) borate de lithium (LiBOB), le nitrate de lithium (LiNOs), le chlorure de lithium (LiCI), le bromure de lithium (LiBr), le fluorure de lithium (LiF), le perchlorate de lithium (LiClO₄), l'hexafluoroarsénate de lithium (LiAsF₆), le trifluorométhanesulfonate de lithium (LiSO₃CF₃) (LiTf), le fluoroalkylphosphate de lithium Li[PF₃(CF₂CF₃)₃] (LiFAP), le tétrakis(trifluoroacétoxy)borate de lithium Li[B(OCOCF₃)₄] (LiTFAB), le bis(1,2-benzenediolato(2-)-O,O')borate de lithium Li[B(C₆O₂)₂] (LBBB) et leurs combinaisons.

19. Électrolyte selon l'une quelconque des revendications 12 à 18, l'électrolyte comprenant en outre un liant d'électrolyte, de préférence le liant d'électrolyte étant un liant polymère de type polyéther, polymère fluoré (tel que le fluorure de polyvinylidène (PVdF)), ou un liant soluble dans l'eau.

20. Cellule électrochimique comprenant une cathode, un électrolyte et une anode, dans laquelle :
- la cathode comprend un matériau d'électrode tel que défini à l'une quelconque des revendications 7 à 10 ; ou
- la cathode est une électrode positive telle que définie à la revendication 11 ; ou
- l'électrolyte est tel que défini à l'une quelconque des revendications 12 à 19 ; ou
- la cathode est une électrode positive telle que définie à la revendication 11 et l'électrolyte tel que défini à l'une quelconque des revendications 12 à 19.

21. Pile au lithium comprenant une cellule électrochimique telle que définie à la revendication 20.

## Patentansprüche

1. Polymer von Formel I: 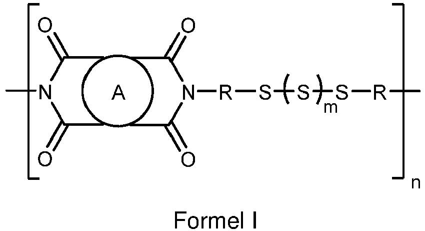 wobei:
A ausgewählt ist aus ungesättigten Gruppierungen, die die Delokalisierung von Elektronen ermöglichen, z. B. substituierten oder unsubstituierten Aryl- und Heteroarylgruppierungen und deren kondensierte oder nicht kondensierte polycyclische Äquivalente, wobei A vorzugsweise ausgewählt ist aus Benzol-, Naphthalin-, Perylen- und Biphenylgruppierungen;
R eine substituierte oder unsubstituierte organische Verbindungsgruppe ist, ausgewählt aus geradkettigem oder verzweigtem C₂₋₆-Alkylen, geradkettigem oder verzweigtem C₂₋₆-Alkylenoxy, linearem oder verzweigtem C₂₋₆-Alkylenglykol, linearem oder verzweigtem C₂₋₆-AlkylenoxyC₂₋₆-Alkylen, linearem oder verzweigtem Poly(C₂₋₆-Alkylenglykol), linearem oder verzweigtem C₆₋₁₂-Arylen, C₃₋₁₂-Cycloalkylen, C₅₋₁₂-Heteroarylen, C₃₋₁₂-Heterocycloalkylen, wobei R vorzugsweise ausgewählt ist aus Benzol-, Ethylen-, Propylen-, Poly(ethylenglycol)-, Poly(propylenglycol)-Gruppierungen und Copolymeren von Ethylenglycol und Propylenglycol;
m die durchschnittliche Anzahl von Schwefelatomen darstellt, die in die Disulfidbrücke der Polymerglieder eingefügt sind, und nicht Null sein kann, d. h. m > 0, z. B. 0 < m ≤ 8, oder 1 ≤ m ≤ 6, oder 1 ≤ m ≤ 4; und
n die durchschnittliche Anzahl der Einheiten in dem Polymer darstellt, z. B. kann n im Bereich von 2 bis 500 oder 5 bis 300 liegen.

2. Polymer nach Anspruch 1, wobei das Polymer von Formel I(a) ist: 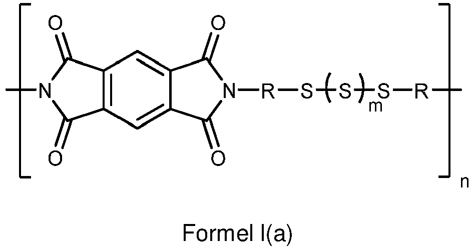 wobei n, R, m und n wie definiert nach Anspruch 1 sind.

3. Polymer nach Anspruch 2, wobei das Polymer von Formel l(b) ist: 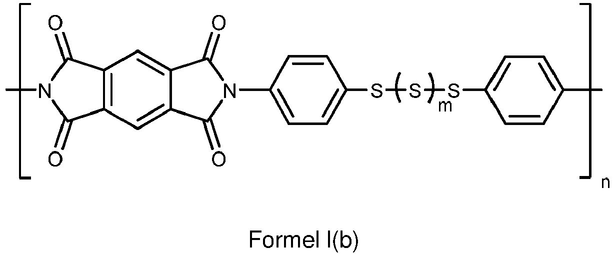 wobei m und n wie definiert nach Anspruch 1 sind.

4. Verbindung von Formel II: 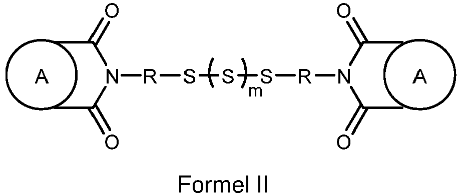 wobei A und R wie definiert in Anspruch 1 sind und m die Anzahl von Schwefelatomen darstellt, die in die Disulfidbrücke der Verbindung eingefügt sind, z. B. m > 0, z. B. 0 < m ≤ 8, oder 1 ≤ m ≤ 6, oder 1 ≤ m ≤ 4.

5. Verbindung nach Anspruch 4, wobei die Verbindung von Formel II(a) ist: 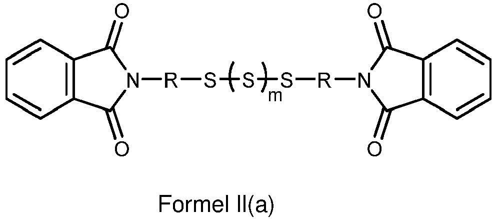 wobei R und m wie definiert in Anspruch 4 sind;

6. Verbindung nach Anspruch 4, wobei die Verbindung von Formel II(b) ist: 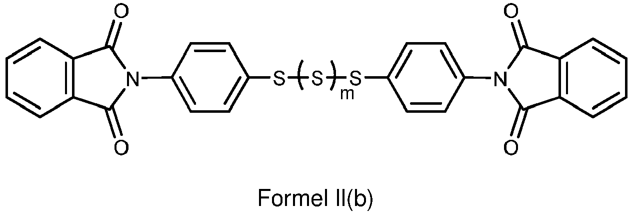 wobei m wie definiert in Anspruch 4 ist.

7. Elektrodenmaterial, umfassend ein Polymer, wie definiert nach einem der Ansprüche 1 bis 3, oder eine Verbindung, wie definiert gemäß einem der Ansprüche 4 bis 6, umfassend optional elementaren Schwefel und/oder ein leitendes Material, ein Bindemittel oder eine Kombination davon.

8. Elektrodenmaterial nach Anspruch 7, wobei das leitende Material ausgewählt ist aus Ruß, Ketjen^{MC}-Kohlenstoff, Shawinigan-Kohlenstoff, Acetylenschwarz, Acetylengas, Graphit, Graphen, Kohlenstofffasern (wie z. B. Kohlenstoffnanofasern (z. B. in der Gasphase gebildetes VGCF)) und Kohlenstoffnanoröhren oder einer Kombination von mindestens zwei davon.

9. Elektrodenmaterial nach Anspruch 7 oder 8, wobei das Bindemittel ein polymeres Bindemittel vom Typ Polyether, Fluorpolymer oder ein wasserlösliches Bindemittel ist.

10. Elektrodenmaterial nach Anspruch 9, wobei:
- das polymere Bindemittel vom Typ Polyether geradlinig, verzweigt und/oder vernetzt ist und auf Poly(ethylenoxid) (PEO), Poly(propylenoxid) (PPO) oder einem Gemisch aus beiden (oder einem EO/PO-Copolymer) basiert und optional vernetzbare Einheiten umfasst; oder
- das Fluorpolymer-Bindemittel PVDF (Polyvinylidenfluorid) oder PTFE (Polytetrafluorethylen) ist; oder
- das wasserlösliche Bindemittel SBR (Styrol-Butadien-Kautschuk), NBR (Acrylnitril-Butadien-Kautschuk), HNBR (hydrierter NBR), CHR (Epichlorhydrin-Kautschuk) oder ACM (Acrylat-Kautschuk), optional einschließlich CMC (Carboxymethylcellulose), ist.

11. Positive Elektrode, umfassend ein Elektrodenmaterial nach einem der Ansprüche 7 bis 10, das auf einen Stromkollektor aufgebracht ist.

12. Elektrolyt, umfassend ein Polymer, wie definiert in einem der Ansprüche 1 bis 3, oder eine Verbindung, wie definiert in einem der Ansprüche 4 bis 6, und optional elementaren Schwefel.

13. Elektrolyt nach Anspruch 12, wobei der Elektrolyt ein flüssiger Elektrolyt ist, umfassend ein Salz in einem Lösungsmittel, oder ein Gelelektrolyt, umfassend ein Salz in einem Lösungsmittel und optional ein solvatisierendes Polymer.

14. Elektrolyt nach Anspruch 13, wobei das Lösungsmittel ein polares aprotisches Lösungsmittel ist, ausgewählt aus Ethylencarbonat (EC), Diethylcarbonat (DEC), Propylencarbonat (PC), Dimethylcarbonat (DMC, Methyl- und Ethylcarbonat (EMC), y-Butyrolacton (y-BL), Vinylencarbonat (VC), Methylbutyrat (MB), y-Valerolacton (y-VL), 1,2-Dimethoxyethan (DME), 1,2-Diethoxyethan (DEE), 2-Methyltetrahydrofuran, Dimethylsulfoxid, Formamid, Acetamid, Dimethylformamid, Dioxolan, Acetonitril, Propylnitril, Nitromethan, Ethylmonoglyme, Trimethoxymethan, Dioxolanderivate, Sulfolan, Methylsulfolan, Propylencarbonatderivate, Tetrahydrofuran und deren Gemischen.

15. Elektrolyt nach Anspruch 12, wobei der Elektrolyt ein fester Polymerelektrolyt (SPE) ist, umfassend ein Salz in einem solvatisierenden Polymer.

16. Elektrolyt nach einem der Ansprüche 12 bis 15, wobei das Polymer oder die Verbindung ein Additiv ist.

17. Elektrolyt nach Anspruch 15, wobei das solvatisierende Polymer das Polymer wie definiert in einem der Ansprüche 1 bis 3 ist, oder die Verbindung, wie definiert in einem der Ansprüche 4 bis 6 ist.

18. Elektrolyt nach einem der Ansprüche 13 bis 17, wobei das Salz ein Lithiumsalz ist, vorzugsweise ausgewählt aus Lithiumhexafluorphosphat (LiPF₆), Lithium-bis(trifluormethyl)tisulfidthansulfonyl)imid (LiTFSI), Lithium-bis(fluorsulfonyl)imid (LiFSI), Lithium-2-trifluormethyl-4,5-dicyano-imidazolat (LiTDI), Lithium-4,5-dicyano-1,2,3-triazolat (LiDCTA), Lithium-bis-(pentafluorethylsulfonyl)-imid (LiBETI), Lithiumtetrafluoroborat (LlBF₄), Lithium-bis-(oxalato)-borat (LiBOB), Lithiumnitrat (LiNOs), Lithiumchlorid (LiCl), Lithiumbromid (LiBr), Lithiumfluorid (LiF), Lithiumperchlorat (LiClO₄), Lithiumhexafluoroarsenat (LiAsF₆), Lithiumtrifluormethansulfonat (LISO₃CF₃) (LiTf), Lithiumfluoroalkylphosphat Li[PF₃(CF₂CF₃)₃] (LiFAP), Lithiumtetrakis(trifluoracetoxy)borat Li[B(OCOCF₃)₄] (LiTFAB), Lithium-bis(1,2-benzenediolato(2-)-O,O')borat Li[B(CeO₂)₂] (LBBB) und deren Kombinationen.

19. Elektrolyt nach einem der Ansprüche 12 bis 18, wobei der Elektrolyt ferner ein Elektrolytbindemittel umfasst, vorzugsweise wobei das Elektrolytbindemittel ein polymeres Bindemittel vom Polyethertyp, ein Fluorpolymer (wie Polyvinylidenfluorid (PVdF)) oder ein wasserlösliches Bindemittel ist.

20. Elektrochemische Zelle, umfassend eine Kathode, einen Elektrolyten und eine Anode, wobei:
- die Kathode umfassend ein Elektrodenmaterial, wie definiert in einem der Ansprüche 7 bis 10 ist; oder
- die Kathode eine positive Elektrode ist, wie definiert in Anspruch 11; oder
- der Elektrolyt wie definiert in einem der Ansprüche 12 bis 19 ist; oder
- die Kathode eine positive Elektrode ist, wie definiert in Anspruch 11, und der Elektrolyt wie definiert in einem der Ansprüche 12 bis 19 ist.

21. Lithiumbatterie, umfassend eine elektrochemische Zelle, wie definiert in Anspruch 20 ist.

## Claims

1. Polymer of Formula I: 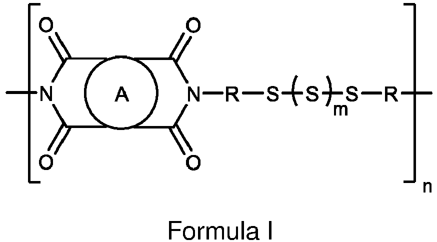 wherein:
A is selected from unsaturated groups allowing the delocalization of electrons, for example, substituted or unsubstituted aryl and heteroaryl groups, and their fused or unfused polycyclic equivalents, preferably A being selected from the benzene, naphthalene, perylene, and biphenyl groups;
R is a substituted or unsubstituted organic linking group selected from linear or branched C₂₋₆alkylene, linear or branched C₂₋₆alkyleneoxy, linear or branched C₂₋₆alkyleneglycol, linear or branched C₂₋₆alkyleneoxyC₂₋₆alkylene, linear or branched poly(C₂₋₆alkyleneglycol), C₆₋₁₂arylene, C₃₋₁₂cycloalkylene, C₅₋₁₂heteroarylene, and C₃₋₁₂heterocycloalkylene, preferably R being selected from the groups benzene, ethylene, propylene, polyethylene glycol), polypropylene glycol), and copolymers of ethylene glycol and propylene glycol;
m represents the average number of sulfur atoms inserted into the disulfide bond of the polymer links and cannot be zero, *i.e.* m > 0, for example, 0 < m ≤ 8, or 1 ≤ m ≤ 6, or 1 ≤ m ≤ 4; and
n represents the average number of units in the polymer, for example, n may be in the range of 2 to 500, or of 5 to 300.

2. Polymer according to claim 1, the polymer being of Formula I(a): 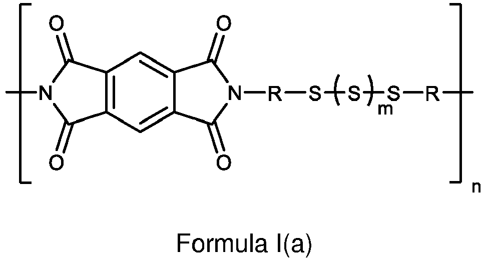 wherein R, m and n are as defined in claim 1.

3. Polymer according to claim 2, the polymer being of Formula I(b): 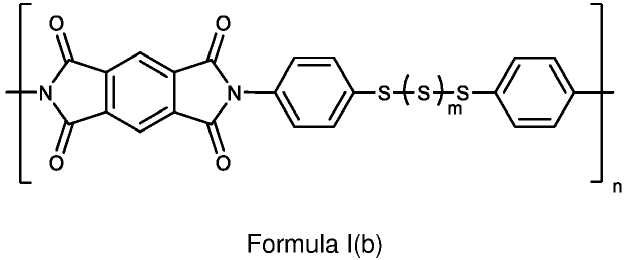 wherein m and n are as defined in claim 1.

4. Compound of Formula II: 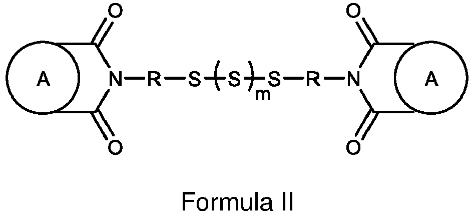 wherein A and R are as defined in claim 1, and m represents the number of sulfur atoms inserted into the disulfide bond of the compound, for example, m > 0, for example, 0 < m ≤ 8, or 1 ≤ m ≤ 6, or 1 ≤ m ≤ 4.

5. Compound according to claim 4, the compound being of Formula II(a): 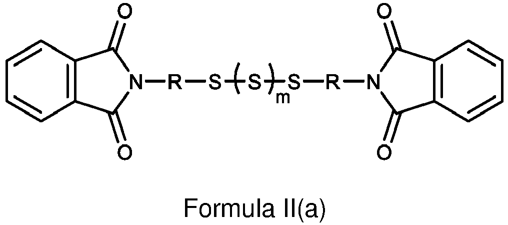 wherein R and m are as defined in claim 4.

6. Compound according to claim 4, the compound being of Formula 11(b): 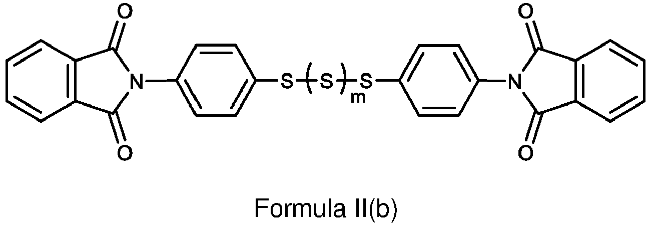 wherein m is as defined in claim 4.

7. Electrode material comprising a polymer as defined in any one of claims 1 to 3 or a compound as defined in any one of claims 4 to 6, and optionally comprising elemental sulfur and/or a conductive material, a binder, or a combination of both.

8. Electrode material according to claim 7, wherein the conductive material is selected from carbon black, Ketjen^{™} carbon, Shawinigan carbon, acetylene black, graphite, graphene, carbon fibers (such as carbon nanofibers (for example, VGCF formed in the gas phase)), and carbon nanotubes, or a combination of at least two thereof.

9. Electrode material according to claim 7 or 8, wherein the binder is a polymeric binder of polyether type, a fluorinated polymer, or a water-soluble binder.

10. Electrode material according to claim 9, wherein:
- the polymeric binder of polyether type is linear, branched and/or crosslinked and is based on polyethylene oxide) (PEO), polypropylene oxide) (PPO), or a mixture of the two (or an EO/PO co-polymer), and optionally comprises crosslinkable units; or
- the fluorinated polymer binder is PVDF (polyvinylidene fluoride) or PTFE (polytetrafluoroethylene); or
- the water-soluble binder is SBR (styrene-butadiene rubber), NBR (acrylonitrile-butadiene rubber), HNBR (hydrogenated NBR), CHR (epichlorohydrin rubber), or ACM (acrylate rubber), optionally comprising CMC (carboxymethyl cellulose).

11. Positive electrode comprising an electrode material according to any one of claims 7 to 10 applied on a current collector.

12. Electrolyte comprising a polymer as defined in any one of claims 1 to 3 or a compound as defined in any one of claims 4 to 6, and optionally elemental sulfur.

13. Electrolyte according to claim 12, the electrolyte being a liquid electrolyte comprising a salt in a solvent or a gel electrolyte comprising a salt in a solvent and optionally a solvating polymer.

14. Electrolyte according to claim 13, the solvent being a polar aprotic solvent selected from ethylene carbonate (EC), diethyl carbonate (DEC), propylene carbonate (PC), dimethyl carbonate (DMC), ethylmethyl carbonate (EMC), γ-butyrolactone (γ-BL), vinylene carbonate (VC), methyl butyrate (MB), γ-valerolactone (γ-VL), 1,2-dimethoxyethane (DME), 1,2-diethoxyethane (DEE), 2-methyltetrahydrofuran, dimethylsulfoxide, formamide, acetamide, dimethylformamide, dioxolane, acetonitrile, propylnitrile, nitromethane, ethylmonoglyme, trimethoxymethane, dioxolane derivatives, sulfolane, methylsulfolane, propylene carbonate derivatives, tetrahydrofuran, and mixtures thereof.

15. Electrolyte according to claim 12, the electrolyte being a solid polymer electrolyte (SPE) comprising a salt in a solvating polymer.

16. Electrolyte according to any one of claims 12 to 15, the polymer or the compound being an additive.

17. Electrolyte according to claim 15, the solvating polymer being the polymer as defined in any one of claims 1 to 3 or the compound as defined in any one of claims 4 to 6.

18. Electrolyte according to any one of claims 13 to 17, wherein the salt is a lithium salt, preferably the salt selected from lithium hexafluorophosphate (LiPF₆), lithium bis(trifluoromethanesulfonyl)imide (LiTFSI), lithium bis(fluorosulfonyl)imide (LiFSI), lithium 2-trifluoromethyl-4,5-dicyanoimidazolate (LiTDI), lithium 4,5-dicyano-1,2,3-triazolate (LiDCTA), lithium bis(pentafluoroethylsulfonyl)imide (LiBETI), lithium tetrafluoroborate (LiBF₄), lithium bis(oxalato)borate (LiBOB), lithium nitrate (LiNOs), lithium chloride (LiCI), lithium bromide (LiBr), lithium fluoride (LiF), lithium perchlorate (LiClO₄), lithium hexafluoroarsenate (LiAsF₆), lithium trifluoromethanesulfonate (LiSOsCFs) (LiTf), lithium fluoroalkylphosphate Li[PF₃(CF₂CF₃)₃] (LiFAP), lithium tetrakis(trifluoroacetoxy)borate Li[B(OCOCF₃)₄] (LiTFAB), lithium bis(1,2-benzenediolato(2-)-O,O')borate Li[B(C₆O₂)₂] (LBBB) and a combination thereof.

19. Electrolyte according to any one of claims 12 to 18, the electrolyte further comprising an electrolyte binder, preferably the electrolyte binder being a polymeric binder of polyether type, a fluorinated polymer (such as polyvinylidene fluoride (PVDF)), or a water-soluble binder.

20. Electrochemical cell comprising a cathode, an electrolyte and an anode, wherein:
- the cathode comprises an electrode material as defined in any one of claims 7 to 10; or
- the cathode is a positive electrode as defined in claim 11; or
- the electrolyte is as defined in any one of claims 12 to 19; or
- the cathode is a positive electrode as defined in claim 11 and the electrolyte is as defined in any one of claims 12 to 19.

21. Lithium battery comprising an electrochemical cell as defined in claim 20.
